# EUROPÄISCHE PATENTSCHRIFT

(11) **EP 2 398 389 B1**
(45) Veröffentlichungstag und Bekanntmachung des Hinweises auf die Patenterteilung: **22.03.2017**
(21) Anmeldenummer: 10705800.0
(22) Anmeldetag: 12.02.2010
(51) Int. Cl.: A61B 5/151, A61B 5/00

(54) **TESTELEMENTMAGAZIN MIT ABGEDECKTEN TESTFELDERN**
Test element magazine with covered test fields
Chargeur d'éléments de test doté de champs de test recouverts

(30) Priorität: 19.02.2009 EP 09153209
(43) Veröffentlichungstag der Anmeldung: 28.12.2011
(60) Teilanmeldung: 16205623.8
(73) Patentinhaber: Roche Diabetes Care GmbH, 68305 Mannheim (DE); F. Hoffmann-La Roche AG, 4070 Basel (CH)
(72) Erfinder: HOENES, Joachim, 64673 Zwingenberg (DE)
(74) Vertreter: Herzog, Fiesser & Partner Patentanwälte PartG mbB
(86) Internationale Anmeldenummer: PCT/EP2010/000866
(87) Internationale Veröffentlichungsnummer: WO 2010/094428

(56) Entgegenhaltungen:
- EP-A- 1 900 321
- EP-A- 1 929 937
- WO-A-01/64105
- WO-A-2005/065414
- WO-A-2006/031920
- WO-A-2008/145625

## Beschreibung

### Gebiet der Erfindung

Die Erfindung betrifft ein analytisches Magazin mit mindestens einem analytischen Hilfsmittel. Derartige analytische Magazine mit analytischen Hilfsmitteln werden insbesondere im Bereich der medizinischen Diagnostik eingesetzt, um einen oder mehrere Analyte in einer Probe einer Körperflüssigkeit nachzuweisen. Insbesondere lassen sich derartige analytische Magazine in integrierten analytischen Systemen einsetzen, welche sowohl eine Probennahmefunktion als auch die genannte Funktion des Nachweises mindestens eines Analyten in einer Körperflüssigkeit bereitstellen. Auch andere Anwendungen sind grundsätzlich möglich, beispielsweise Anwendungen außerhalb der medizinischen Diagnostik. Ohne Beschränkung weiterer möglicher Anwendungen wird die Erfindung im Folgenden im Wesentlichen unter Bezugnahme auf den Nachweis von Blutglukose beschrieben,

### Stand der Technik

Insbesondere aus dem Bereich der medizinischen Diagnostik, jedoch auch aus anderen Bereichen der Naturwissenschaften, der Medizin und der Technik, sind Systeme bekannt, mittels derer sich Analyte in Proben nachweisen lassen. In der medizinischen Diagnostik umfasst ein derartiger Nachweis, beispielsweise von Blutglukose, in der Regel ein Generieren einer Probe von Körperflüssigkeit, beispielsweise Blut oder interstitieller Flüssigkeit, gefolgt von einer Aufnahme dieser Probe und einer qualitativen und/oder quantitativen Analyse. Zu diesem Zweck werden in der Regel ein oder mehrere analytische Hilfsmittel verwendet, welche beispielsweise eine Lanzette und/oder Testelementen umfassen können, mittels derer die Probe generiert und/oder aufgenommen und/oder analysiert werden kann.

Insbesondere die Integration verschiedener Systemfunktionen hat bei derartigen analytischen Systemen, beispielsweise Blutzucker-Messsystemen, in der Vergangenheit zu kommerziellen Lösungen mit auswechselbaren Magazinen für die analytischen Hilfsmittel, beispielsweise Teststeifen-Magazinen, geführt. Typische Vertreter dieser Produktgruppe sind die Systeme Accu-Chek Compact® sowie Ascensia Breeze, welche kommerziell erhältliche Systeme darstellen. Typische zugehörige Magazine fassen in der Regel 17 bzw. 10 Teststeifen.

In neueren Systemen werden dabei in vielen Fällen unterschiedliche Systemfunktionen integriert zusammengefasst, beispielsweise die Systemfunktion einer Probengenerierung (beispielsweise durch Perforation einer Hautpartie) und die Systemfunktion einer Probenaufnahme sowie optional auch die Systemfunktion einer Analyse. Bei der Konzeption höher integrierter Systeme können beispielsweise eine Blutgewinnung und eine Testfunktion zusammengeführt werden. Zu diesem Zweck sind beispielsweise so genannte "Microsampler" bekannt, welche die Funktion einer Lanzette und die Funktion eines Transports der Probe, beispielsweise hin zu einem Testelement, zusammenfassen können. Eine separate Stechhilfe zur Blutentnahme, beispielsweise aus einer Fingerbeere, einem Ohrläppchen oder einer sonstigen Hautpartie eines Probanden, kann somit eingespart werden.

Allgemein können mehrere analytische Hilfsmittel, beispielsweise mehrere Microsampler, in einem fest integrierten Magazin oder in einem Wechselmagazin unterschiedlichster Bauart in einem analytischen System untergebracht werden. Grundsätzlich lassen sich, unabhängig von der Art des analytischen Hilfsmittels, drei Haupttypen von analytischen Magazinen unterscheiden, nämlich Rundmagazine (beispielsweise in Form von Trommeln und/oder Scheiben), Linearmagazine (beispielsweise in Form von Stapelmagazinen, ZickZack-Magazinen oder ähnlichem) und Bandmagazine, bei welchen die analytischen Hilfsmittel auf einem Band oder einer anderen Form von zumindest teilweise flexiblem Träger angeordnet sind. Diese Magazintypen lassen sich grundsätzlich auch im Rahmen der im Folgenden beschriebenen Erfindung einsetzen, bzw. modifizieren. Im Stand der Technik werden Rundmagazine beispielsweise in US 2006/0008389, US 2007/0292314, US 2006/0184064, US 2003/0212347 oder US 2002/0087056 beschrieben. Linearmagazine sind beispielsweise in US 6,036,924 oder in US 2003/0191415 beschrieben. Bandmagazine werden beispielsweise offenbart in US 2002/0188224, in US 2008/0103415, in EP 1360935 A1 oder in DE 19819407 A1.

Neben getrennten Magazinen für Lanzetten und Testelemente, welche eine Testchemie enthalten, setzen sich im Rahmen der oben beschriebenen Integration, also Magazine durch, in welchen sowohl Testelemente als auch Lanzetten aufgenommen sind, beispielsweise in Form so genannter Kombi-Disposables. Diese Hilfsmittel können in einer gewünschten Anzahl in dem Magazin magaziniert werden, und das Magazin kann in einem entsprechenden Gerät, welches im Folgenden auch als analytisches System bezeichnet wird, eingesetzt werden. Derartige Konstruktionen führen jedoch in der Regel zu eher komplexen, großen Systemen. Auch die Unterbringung von Testelementen, beispielsweise Testfeldern und Lanzetten auf einem Band, beispielsweise im Rahmen so genannter "Lancet-on-tape"-Systeme, behält den Nachteil, dass funktionsrelevante Teile bei einer Herstellung zunächst vereinzelt und dann wieder auf ein Band aufgebracht werden müssen.

Eine weitere technische Herausforderung von Kombimagazinen, bei welchen analytische Hilfsmittel magaziniert werden, die sowohl Lanzetten als auch Testelemente, insbesondere mit einer Testchemie, umfassen, besteht in den hohen Anforderungen hinsichtlich der Biokompatibilität der analytischen Hilfsmittel. Insbesondere kann die in den Testelementen verwendete Testchemie beispielsweise Enzyme und/und andere Materialien, beispielsweise Hilfsstoffe, umfassen, welche bei direktem Kontakt mit Körpergewebe eines Probanden zu Unverträglichkeiten führen können. Obwohl derartige Phänomene noch nicht in allen Einzelheiten untersucht sind, wäre es beispielsweise denkbar, dass bestimmte Enzyme allergische Reaktionen im Probanden auslösen. Es ist daher auch bei integrierten Systemen und entsprechenden Magazinen für integrierte Systeme darauf zu achten, dass, neben einer einfachen und kostengünstigen Herstellung, die Biokompatibilität derartiger Systeme in jedem Fall gewährleistet ist.

So offenbaren beispielsweise WO 2006/031920 A2 und WO 2005/065414 A2 jeweils Systeme und Verfahren für eine Probennahme. Dabei durchdringt ein Stechelement jeweils ein Testelement, bevor das Stechelement in eine Hautpartie eines Benutzers eindringt. Beim Zurückziehen des Stechelements wird die an dem Stechelement anhaftende Probe von außen auf das Testelement aufgebracht. Nachteilig dabei ist jedoch, dass bereits bei der Vorwärtsbewegung des Stechelements, also beim Stechvorgang, beim Durchdringen des Testelements Teile der Testchemie an dem Stechelement anhaften könnten und somit zu einem unerwünschten Eintrag von Testchemie in den Körper des Probanden führen könnten.

In ähnlicher Weise offenbart WO 01/64105 A1 eine Vorrichtung zur Detektion und Mengenbestimmung eines Analyten. Auch bei dieser Vorrichtung ist vorgesehen, dass eine Lanzette bei einem Probennahmevorgang ein Testfeld durchdringt und beim Zurückziehen aus dem Körpergewebe des Benutzers Körperflüssigkeit von außen an dem Testfeld abstreift. Auch in dieser Anordnung treten die oben beschriebenen Probleme einer Möglichkeit eines Eintrags von Testchemie in das Körpergewebe auf.

Weiterhin tritt bei den genannten Vorrichtungen oder bei anderen bekannten Vorrichtungen häufig das Problem auf, dass insbesondere Kanten der Testfelder zum Zerfall neigen können. Auf diese Weise können beispielsweise von den Kanten der Testfelder Bestandteile der Testchemie abbröckeln und zur Lanzette gelangen.

### Aufgabe der Erfindung

Es ist daher eine Aufgabe der vorliegenden Erfindung, ein analytisches Magazin bereitzustellen, welches die genannten technischen Herausforderungen bekannter Magazine löst. Insbesondere soll das analytische Magazin auch für den Einsatz in integrierten Systemen geeignet sein und soll kostengünstig herstellbar sein, bei gleichzeitiger Gewährleistung der beschriebenen Biokompatibilität.

### Offenbarung der Erfindung

Diese Aufgabe wird durch die Erfindung mit den Merkmalen der unabhängigen Ansprüche gelöst. Vorteilhafte Weiterbildungen der Erfindung, welche einzeln oder in Kombination realisierbar sind, sind in den abhängigen Ansprüchen dargestellt.

Die vorliegende Erfindung geht aus von der Erkenntnis, dass während der Lagerung analytischer Hilfsmittel, welche sowohl eine Lanzette als auch ein Testelement umfassen können, innerhalb der gleichen Magazinkammer eines analytischen Magazins unter Umständen Kontaminationen der Lanzette, beispielsweise einer Nadelspitze, durch Testchemie des Testelements auftreten können. Somit können, wie oben dargestellt, beispielsweise Teile der Testchemie während eines Stechvorgangs in die Haut eines Patienten eindringen. Ausgehend von dieser Erkenntnis beruht die im Folgenden dargestellte Erfindung wesentlich auf dem Gedanken, dass im trockenen Zustand der Testchemie eine Kontamination weitere Bestandteile der analytischen Hilfsmittel, beispielsweise einer Lanzette, insbesondere durch partikuläre Bestandteile der Testchemie verursacht werden können. Derartige partikuläre Bestandteile, beispielsweise Partikel, Partikelkonglomerate oder "Krümel" können auch bei sorgfältigster Herstellung durch verschiedene Prozesse entstehen. So werden beispielsweise Testfelder mit der entsprechenden Testchemie in vielen Fällen durch Schneideverfahren hergestellt, was unten näher ausgeführt wird. An Schneidekanten können derartige entsprechende partikuläre Verunreinigungen auftreten. Weiterhin können Partikel auch im Rahmen des Betriebs der analytischen Magazine freigesetzt werden, beispielsweise durch Erschütterungen oder mechanische Abriebe. Insbesondere kann dies dann geschehen, wenn Testfelder mit weiteren Elementen in Berührung kommen, beispielsweise weiteren Teil-Hilfsmitteln, insbesondere den genannten Lanzetten selbst.

Die nachfolgend näher dargestellte Erfindung beruht auf dem gemeinsamen Erfindungsgedanken, derartige Kontaminationen, insbesondere Kontaminationen durch partikuläre Verunreinigungen, durch entsprechende Schutzmaßnahmen zu vermeiden. Insbesondere soll erfindungsgemäß verhindert werden, dass derartige Verunreinigungen auf optionale Lanzetten übertragen werden können. Dementsprechend können ein oder mehrere Elemente vorgesehen sein, welche als Trennelemente wirken und welche eingerichtet sind, um einerseits eine Übertragung von Verunreinigungen von Testelementen auf andere Teile der analytischen Hilfsmittel, insbesondere in derselben Kammer gelagerten Lanzetten, insbesondere den Übertrag von Partikeln, zumindest weitgehend zu verhindern und welcher andererseits einen zumindest teilweisen Zugang der Probe zu einer Testchemie des Testelements ermöglichen, beispielsweise einen Zugang einer Blutprobe und/oder von Bestandteilen dieser Blutprobe zur Testchemie.

Ausgehend von diesem gemeinsamen Konzept wird in einem ersten Aspekt, welcher vorliegend jedoch nicht separat beansprucht wird, ein analytisches Magazin vorgeschlagen, welches mindestens eine Kammer mit mindestens einem analytischen Hilfsmittel umfasst. Das analytische Hilfsmittel umfasst mindestens eine Lanzette.

Unter einem analytischen Magazin wird dabei allgemein eine Vorrichtung, vorzugsweise eine kompakte, insgesamt einstückig ausgebildete Vorrichtung, verstanden, welche für den Einsatz in einem analytischen System zum Nachweis mindestens eines Analyten in einer Probe einer Körperflüssigkeit eingesetzt werden kann. Beispielsweise kann auf die oben dargestellten Anwendungsbeispiele verwiesen werden. Entsprechend kann das analytische Magazin beispielsweise eine der bekannten Magazinformen aufweisen. So kann das analytische Magazin beispielsweise als Rundmagazin, insbesondere als Trommelmagazin und/oder als Scheibenmagazin, als Bandmagazin oder als Stangenmagazin ausgestaltet sein. Bei dem Analyten kann es sich insbesondere um mindestens einen Metaboliten und/oder eine andere physikalisch und/oder chemisch nachweisbare Eigenschaft der Probe der Körperflüssigkeit handeln, wie beispielsweise Blutglukose, Cholesterin, Koagulate oder ähnliches.

Unter einer Kammer ist allgemein eine Aufnahme zu verstehen, in welcher mindestens ein analytisches Hilfsmittel vollständig oder teilweise aufgenommen ist. Die Kammer kann dabei mindestens einen Innenraum aufweisen, wobei auch mehrere Innenräume, welche zusammenhängend ausgestaltet sein können oder getrennt ausgestaltet sein können, vorgesehen sein können. Die mindestens eine Kammer kann geschlossen ausgestaltet sein, kann jedoch auch zumindest teilweise geöffnet sein, also mindestens eine Öffnung aufweisen. Die Kammer kann beispielsweise in einem Gehäuse und/oder einem Gehäuseteil des analytischen Magazins ausgebildet sein, wobei Kammerwände in dem Gehäuse ausgebildet sein können, welche die Kammern begrenzen und/oder definieren. Das analytische Magazin kann eine oder vorzugsweise mehrere derartiger Kammern umfassen. Besonders bevorzugt sind analytische Magazine mit mindestens 10, vorzugsweise mindestens 20, mindestens 25 oder sogar mehr als 50 Kammern.

Unter einem analytischen Hilfsmittel kann dabei ein beliebiges Hilfsmittel verstanden werden, welches für den qualitativen und/oder quantitativen Nachweis des mindestens einen Analyten in der Probe der Körperflüssigkeit eingesetzt werden kann und/oder einen derartigen Nachweis unterstützen kann. Vorzugsweise sind, wenn mehrere Kammern vorgesehen sind, in jeder Kammer mindestens eines der analytischen Hilfsmittel aufgenommen. Jedes dieser analytischen Hilfsmittel kann sich wiederum aus mehreren Teil-Hilfsmitteln zusammensetzen. Die Aufnahme der analytischen Hilfsmittel in den Kammern kann vollständig oder teilweise erfolgen, so dass beispielsweise auch Teile der analytischen Hilfsmittel außerhalb der Kammern angeordnet sein können, beispielsweise Teil-Hilfsmittel der analytischen Hilfsmittel. Insbesondere soll vom Begriff der Aufnahme in einer Kammer auch der Fall erfasst sein, dass die analytischen Hilfsmittel ganz oder teilweise in Kammerwänden der Kammern, insbesondere in dem Gehäuse und/oder einem Gehäuseteil, integriert sind bzw. mit diesem Gehäuse bzw. Gehäuseteil verbunden sind, wobei diese vom Innenraum der Kammer aus zugänglich sein sollten.

Bei dem vorgeschlagenen analytischen Magazin umfasst das mindestens eine analytische Hilfsmittel jeweils mindestens eine Lanzette. Beispielsweise kann diese Lanzette als einfache Lanzettennadel oder Lanzettenschneide ausgestaltet sein. Besonders bevorzugt ist es jedoch, wenn die mindestens eine Lanzette weiterhin mindestens ein Sammelelement zum Sammeln und/oder Transportieren der Probe umfasst, insbesondere ein Kapillarelement. Dementsprechend kann die mindestens eine Lanzette insbesondere ganz oder teilweise als Microsampler ausgestaltet sein. Darüber hinaus kann das mindestens eine analytische Hilfsmittel weitere Arten analytischer Hilfsmittel und/oder Teil-Hilfsmittel umfassen. So kann das analytische Hilfsmittel weiterhin eines oder mehrere der folgenden Hilfsmittel umfassen: ein Transferelement zum Transferieren einer Probe von einer Haut eines Probanden auf ein Testelement; ein Testelement mit mindestens einer Testchemie. Das analytische Hilfsmittel kann dabei, wie oben beschrieben, aus mehreren Teil-Hilfsmitteln zusammengesetzt sein, welche miteinander verbunden sein können, welche jedoch auch unabhängig voneinander ausgestaltet sein können, beispielsweise unabhängig voneinander angeordnet sein können. So kann beispielsweise mindestens ein Teil-Hilfsmittel in der Kammer beweglich gelagert sein, wohingegen mindestens ein weiteres Teil-Hilfsmittel in der Kammer im Wesentlichen ortsfest aufgenommen ist. Beispielsweise kann eine beweglich gelagerte Lanzette als Teil-Hilfsmittel vorgesehen sein, und ein im Wesentlichen unbeweglich gelagertes Testelement.

Unter eine Lanzette ist dabei allgemein ein Element zu verstehen, welches eingerichtet ist, um mindestens eine Öffnung, beispielsweise einen Einstich und/oder einen Einschnitt, in einem Gewebe eines Probanden, beispielsweise einer Haut eines Probanden, zu erzeugen. Zu diesem Zweck kann die Lanzette beispielsweise eine Spitze und/oder eine Schneide umfassen. Insbesondere kann die Lanzette als Rundlanzette und/oder als Flachlanzette ausgestaltet sein, letztere beispielsweise durch entsprechende Herstellung aus einem Metallband, beispielsweise mittels eines Ätzverfahrens.

Unter einem Microsampler ist eine Kombination einer Lanzette mit einem Kapillarelement, beispielsweise einer Kapillare und/oder einem Kapillarspalt, zu verstehen, wobei das Kapillarelement eingerichtet ist, um unter Ausnutzung von Kapillarkräften die Probe zumindest teilweise aufzunehmen und/oder zu transferieren. Insofern kann das Kapillarelement beispielsweise auch vollständig oder teilweise als Transferelement wirken. Alternativ oder zusätzlich können auch andere Arten von Transfer- oder Transportelementen vorgesehen sein, beispielsweise bewegliche Transferelemente oder ähnliches.

Unter einem Testelement ist ein Element zu verstehen, welches mindestens eine Testchemie aufweist, mittels derer der qualitative und/oder quantitative Nachweis des mindestens einen Analyten geführt werden kann. Insbesondere kann die Testchemie ausgestaltet sein, um bei Anwesenheit des mindestens eines Analyten eine oder mehrere physikalisch und/oder chemisch nachweisbare Eigenschaften zu ändern, beispielsweise eine optisch messbare Eigenschaft, insbesondere eine Farbeigenschaft, und/oder eine elektrochemisch nachweisbare Eigenschaft. Bezüglich möglicher Ausgestaltungen der Testchemie kann beispielsweise auf J. Hoenes et al., Diabetes Technology & Therapeutics, Volume 10, Supplement 1, 2008, S-10 - S-26, verwiesen werden. Weiterhin kann beispielsweise auf WO 2007/012494 A1 verwiesen werden. Dort werden besonders feuchtigkeitsstabile Testchemien beschrieben. Die in diesen Druckschriften genannten Testchemien können, einzeln oder in Kombination, auch im Rahmen der vorliegenden Erfindung eingesetzt werden. Insbesondere können stark spezifische Testchemien eingesetzt werden, bei welchen der Nachweis spezifisch auf den mindestens einen Analyten reagiert.

Das Testelement umfasst mindestens ein Testfeld mit mindestens einer Testchemie, welche zumindest teilweise innerhalb der Kammer angeordnet ist. Das Testfeld kann beispielsweise in die Kammern und/oder das Magazin, beispielsweise eine Kammerwand und/oder ein Gehäuse, integriert sein. So kann das Testfeld beispielsweise einen Teil der Kammerwand bilden, also die Kammer in zumindest einer Richtung begrenzen.

Unter einer Testchemie ist allgemein ein Material zu verstehen, welches, wie oben beschrieben, bei Anwesenheit des mindestens einen nachzuweisenden Analyten mindestens eine nachweisbare Eigenschaft ändert, beispielsweise eine physikalische und/oder chemische Eigenschaft. Diese Eigenschaftsänderungen können insbesondere spezifisch auftreten, das heißt ausschließlich bei Anwesenheit des mindestens einen nachzuweisenden Analyten, nicht hingegen oder lediglich in erheblich schwächerem Maße bei Anwesenheit anderer Substanzen. Dabei können jedoch Eigenschaftsänderungen toleriert werden, die bei Anwesenheit von Substanzen auftreten, die mit hoher Wahrscheinlichkeit nicht in der Probe enthalten sind oder lediglich in vernachlässigbarer Konzentration.

Unter dem Begriff eines Testfeldes ist eine flächig aufgebrachte, zusammenhängende Menge der Testchemie zu verstehen. Beispielsweise kann das Testfeld eine Schicht oder einen Bereich einer Schicht der Testchemie umfassen, welche beispielsweise auf einen Träger aufgebracht sein können. Beispielsweise kann die Schicht als geschlossene Schicht ausgestaltet sein, welche selbst auch aus einer oder mehreren Teil-Schichten zusammengesetzt sein kann. Die Schicht kann beispielsweise eine konstante Dicke aufweisen. Die Schicht kann geschlossen sein oder kann auch eine oder mehrere Öffnungen aufweisen. Der optionale Träger selbst ist dabei jedoch nicht Bestandteil des Testfeldes, sondern der Begriff des Testfeldes bezieht sich ausschließlich auf die Testchemie und gegebenenfalls mit der Testchemie verbundene Hilfsstoffe und/oder Hilfsschichten, wie beispielsweise eine optionale Abtrennschicht oder ähnliches. Das Testfeld stellt also die Menge des Materials bereit, welche an dem tatsächlichen Nachweis beteiligt ist und nicht beispielsweise ausschließlich einer mechanischen Stabilisierung dienende Elemente wie beispielsweise einen Träger.

Unter einer Testfeldfläche ist die Oberfläche des Testfeldes zu verstehen, welche für eine Aufgabe der Probe zugänglich ist. Ist beispielsweise das Testfeld auf einen Träger aufgebracht, so kann die Probenaufgabe beispielsweise auf der von dem Träger wegweisenden Oberfläche des Testfeldes ermöglicht sein, so dass diese Oberfläche als Probenaufgabefläche und damit als Testfeldfläche genutzt werden kann. Wie unten noch näher ausgeführt wird, kann diese Oberfläche jedoch räumlich durch zusätzliche Elemente, beispielsweise einen Rahmen, vollständig oder teilweise bedeckt sein, so dass lediglich ein Teil dieser Oberfläche für eine Probenaufgabe zugänglich sein kann. Lediglich dieser zugängliche Teil ist dann als Testfeldfläche zu bezeichnen. Dies kann beispielsweise, wie unten noch näher erläutert wird, durch eine Wand der Kammer erfolgen, welche eine oder mehrere Öffnungen aufweist, wobei eine Probenaufgabe von der Kammer aus auf das Testfeld durch die Öffnung hindurch erfolgt, so dass lediglich der durch die Öffnung hindurch von der Kammer aus sichtbare Bereich des Testfeldes oder die Oberfläche dieses Bereichs als Probenaufgabefläche und damit als Testfeldfläche dient. Dies wird unten exemplarisch noch näher erläutert.

Unter dem Begriff eines Testelements ist somit allgemein ein Element zu verstehen, welches mindestens ein Testfeld mit mindestens einer Testchemie aufweist. Das Testelement kann optional beispielsweise einen oder mehrere Träger umfassen, beispielsweise eine Trägerfolie oder ähnliches, beispielsweise einen Träger aus einem Kunststoffmaterial, einem Papiermaterial, einem keramischen Material oder einer Kombination der genannten und/oder anderer Materialien. Auch Testelemente mit Testfeldern in Form von freistehenden Testchemie-Filmen sind jedoch grundsätzlich denkbar.

Wie oben dargestellt, soll der Begriff einer Anordnung innerhalb der Kammer allgemein umfassen, dass das Testfeld, insbesondere dessen Testfeldfläche, von der Kammer aus zugänglich ist, insbesondere für die Probe der Körperflüssigkeit und/oder Bestandteile der Probe. Unter einer Zugänglichkeit ist insbesondere zu verstehen, dass Probe im Inneren der Kammer und/oder von dem Inneren der Kammer aus auf das Testfeld aufgebracht werden kann. Das Testfeld und insbesondere mindestens eine Testfeldfläche dieses Testfeldes, also eine Fläche, auf welche Probe aufgebracht werden kann, soll also derart mit dem Inneren der Kammer in Verbindung stehen, dass vom Inneren der Kammer aus Probe auf die Testfeldfläche aufgebracht werden kann, im Gegensatz beispielsweise zu einer Aufgabe der Probe von außen, außerhalb der Kammer. Dieses Aufbringen der Probe kann insbesondere durch einen direkten Übertrag der Probe von der Lanzette auf die Testfeldfläche erfolgen. Beispielsweise kann zu diesem Zweck die Lanzette nach einem Stechvorgang zumindest vorübergehend wieder derart in der Kammer aufgenommen sein, dass eine derartige räumliche Nähe zwischen der aufgenommenen Probe, welche beispielsweise unmittelbar an der Lanzette und/oder in einer optionalen, mit der Lanzette verbundenen Kapillare, beispielsweise einem Kapillarspalt, aufgenommen sein kann, besteht, dass zumindest teilweise ein Übertrag der Probe auf die Testfeldfläche erfolgen kann. Alternativ oder zusätzlich können jedoch auch Hilfsmittel vorgesehen sein, beispielsweise innerhalb des analytischen Magazins und/oder in einem das analytische Magazin verwendenden analytischen System, welche den Übertrag der Probe von der Lanzette auf die Testfeldfläche erleichtern. Beispielsweise kann mindestens ein Aktor vorgesehen sein, welcher vorübergehend die Lanzette und/oder den Kapillarspalt auf die Testfeldfläche drückt oder in deren Nähe bringt. Wiederum alternativ oder zusätzlich können beispielsweise die Kammerwände gekrümmt ausgestaltet sein, um ein Durchbiegen der Lanzette derart zu gewährleisten, dass der Übertrag erfolgen kann. Verschiedene Ausgestaltungen sind möglich.

Das Testfeld kann beispielsweise vollständig innerhalb der Kammer gelagert sein oder auch ganz oder teilweise in ein Gehäuse des analytischen Magazins integriert sein und/oder in ein Gehäuseteil dieses Gehäuses. Für die Herstellung der Zugänglichkeit zu dem Testfeld kann beispielsweise, wie oben bereits teilweise erläutert und wie unten noch näher ausgeführt wird, eine Öffnung und/oder eine Ausbuchtung und/oder ein Sackloch in einer Kammerwand und/oder einem Gehäuse des analytischen Magazin vorgesehen sein, durch welche die Probe der Körperflüssigkeit und/oder Bestandteile der Probe der Körperflüssigkeit zu dem Testfeld gelangen können. Die mindestens eine Öffnung kann beispielsweise mindestens eine durchgängige Öffnung in der Wand der Kammer umfassen, also eine Öffnung, durch welche von außerhalb des Magazins, sofern das Testfeld nicht auf diese Öffnung aufgebracht ist, das Innere der Kammer zugänglich ist.

Es wird vorgeschlagen, dass eine Wand der Kammer das Testfeld zumindest teilweise überdeckt und dadurch eine von der Kammer aus zugängliche Testfeldfläche des Testfeldes zur Probenaufgabe zumindest teilweise begrenzt. Unter einer Begrenzung ist dabei allgemein eine Anordnung zu verstehen, bei welchem in zumindest einer Dimension, vorzugsweise in zwei Dimensionen, die Testfeldfläche unmittelbar an die Wand der Kammer angrenzt. Weist beispielsweise das Testelement mindestens einen Träger auf, auf welchen das mindestens eine Testfeld mit der mindestens einen Testchemie aufgebracht ist, so kann beispielsweise, wie oben ausgeführt, eine von dem Träger wegweisende Oberfläche des Testfeldes, einschließlich eines möglicherweise auf diese Oberfläche aufgebrachten Verteilerelements und/oder Abtrennelements, für eine Probenaufgabe benutzt werden. Bei einer Begrenzung durch die Wand der Kammer, wie sie vorliegend vorgeschlagen wird, wird diese Oberfläche jedoch in mindestens einer Dimension durch die Wand der Kammer begrenzt, beispielsweise indem diese Oberfläche teilweise von der Wand der Kammer bedeckt ist und teilweise freiliegt. Der freiliegende Bereich, welcher dann noch für eine Aufgabe der Probe von der Kammer aus zugänglich ist, ist dann die Testfeldfläche im Sinne der vorliegenden Erfindung. Wie oben ausgeführt, kann dies beispielsweise eine TeilFläche der Oberfläche sein, welche von der Kammer aus durch eine Öffnung in der Kammerwand sichtbar und für eine Probenaufgabe zugänglich ist.

Hierdurch unterscheidet sich unter anderem das vorgeschlagene Magazin beispielsweise von der in WO 2006/031920 A2 und WO 2005/065414 A2 beschriebenen Anordnung, bei welcher einen Probenauftrag von außen erfolgt, indem beim Zurückziehen des Stechelements von außen her ein Übertrag der Probe auf ein Gewebe erfolgt, welches anschließend für eine Verteilung auf Nachweisflächen sorgt. Bei dem offenbarten Magazin hingegen kann ein Auftrag der Probe vom Inneren der Kammer her erfolgen. Die Testchemie und die Lanzette können während des gesamten Probennahmevorganges räumlich getrennt sein, so dass ein Eintrag von Testchemie in das Körpergewebe des Benutzers vollständig vermieden werden kann. Gleichzeitig ermöglicht, wie unten noch näher ausgeführt wird, die mögliche Integration des Testfeldes in die Kammerwand eine stark vereinfachte Herstellung.

Die Begrenzung der Testfeldfläche kann beispielsweise dadurch erfolgen, dass die Wand der Kammer entlang einer oder mehrerer Begrenzungslinien unmittelbar auf der Testfeldfläche aufliegt oder derart in der Nähe der Testfeldfläche liegt, dass ein von der Wand unbedeckter Bereich der Testfeldfläche für die Probe zugänglich ist, ein von der Wand bedeckter Bereich hingegen nicht. Die Begrenzungslinie kann sich dabei um die gesamte von der Kammer aus zugängliche Testfeldfläche herum erstrecken, so dass die Begrenzungslinie den Umfang der von der Kammer aus zugänglichen Testfeldfläche vollständig definiert. Auch eine lediglich teilweise Definition des Umfangs der von der Kammer aus zugänglichen Testfeldfläche durch die Wand der Kammer ist möglich, wobei die übrige Begrenzung der von der Kammer aus zugänglichen Testfeldfläche beispielsweise durch eine laterale Begrenzung der Testfeldfläche selbst erfolgen kann, beispielsweise einen Rand einer auf einen Träger aufgedruckten Testfeldfläche, welcher von der Wand der Kammer nicht oder nur teilweise bedeckt ist.

Vorzugsweise kann die von der Kammer aus zugängliche Testfeldfläche durch die Kammerwand vollständig definiert werden, so dass beispielsweise die Kammerwand die von der Kammer aus zugängliche Testfeldfläche vollständig definiert. Dies kann beispielsweise dadurch erfolgen, dass, wie oben dargestellt, in der Wand der Kammer eine oder mehrere Öffnungen vorgesehen sind, welche im Folgenden auch als "Fenster" bezeichnet werden und welche grundsätzlich beliebige Gestalt aufweisen können. Besonders bevorzugt sind rechteckige oder runde Formen der Fenster. Alternativ können die Kammern und/oder ein Gehäuse des analytischen Magazins auch eine Ausbuchtung und/oder ein Sackloch aufweisen, beispielsweise in einer Kammerwand und/oder einem anderen Teil des Gehäuses. Insofern ist der Begriff der Kammerwand weit zu fassen und kann grundsätzlich, neben Elementen, welche die tatsächlichen Dimensionen der Kammern begrenzen, auch andere Elemente der Kammern und/oder des Gehäuses umfassen, welche den Zugang zu dem Testfeld begrenzen können.

Durch die zumindest teilweise Überdeckung des Testfeldes durch die Wand der Kammer können insbesondere besonders gefährdete Bereiche des Testfeldes abgedeckt werden, so dass eine Kontamination, welche von diesen Bereichen ausgehen kann, zumindest weitgehend verhindert werden kann. Wie oben dargestellt, kann es sich bei diesen Bereichen insbesondere um Schneidekanten handeln. Dementsprechend kann das Testfeld insbesondere mindestens eine Schneidekante aufweisen, wobei die Wand der Kammer die Schneidekante zumindest teilweise überdeckt. Insbesondere können die oben beschriebenen Fenster derart ausgestaltet sein, dass die Schneidekante nicht Bestandteil der von der Kammer aus zugänglichen Testfeldfläche ist, wobei sinngemäß auch mehrere derartiger Testfeldflächen vorgesehen sein können. In anderen Worten kann insbesondere die Schneidekante derart überdeckt werden, dass diese von der Kammer aus nicht zugänglich ist, so dass umgekehrt beispielsweise partikuläre Verunreinigungen von dieser Schneidekante nicht oder nur erschwert in die Kammer gelangen können, um dort beispielsweise weitere Bestandteile des analytischen Hilfsmittels, beispielsweise mindestens eine Lanzette, zu kontaminieren.

Unter einer Schneidekante ist dabei allgemein eine Grenze des Testfelds zu verstehen, welche beispielsweise durch ein räumlich strukturiertes Druckverfahren und/oder ein Schneideverfahren erzeugt werden kann. Das Testfeld kann beispielsweise derart ausgestaltet sein, dass dieses mindestens einen Träger umfasst, auf welchen die mindestens eine Testchemie aufgebracht ist, beispielsweise in Form einer oder mehrerer Schichten. Der Träger kann beispielsweise, wie unten noch näher ausgeführt wird, beispielsweise ein Kunststoffmaterial und/oder ein Papiermaterial und/oder ein anderes Trägermaterial, welches zur Aufnahme der mindestens einen Testchemie geeignet ist, ausgestaltet sein. Alternativ können jedoch auch so genannte freistehende Testchemie-Filme eingesetzt werden.

Die Ränder des Testfeldes können beispielsweise derart hergestellt werden, dass die Testchemie beispielsweise bereits strukturiert auf den Träger aufgebracht wird. In diesem Falle sind die Grenzen der Strukturierung, beispielsweise der auf den Träger aufgedruckten Testfelder, unter den Begriff der Schneidekante zu subsumieren. Bevorzugt ist jedoch, was alternativ oder zusätzlich realisiert werden kann, eine Begrenzung der Testfelder durch einen tatsächlichen Schneideprozess, beispielsweise mittels eines mechanischen Schneidevorgangs mit mindestens einer Klinge und/oder einem Schneiderad oder einem ähnlichen Schneideelement und/oder ein anders ausgestalteter Schneidevorgang, insbesondere ein Laserschneiden oder ähnliches. Insbesondere kann bei diesem Schneidevorgang das Testfeld der mindestens einen Testchemie gleichzeitig mit dem optionalen Trägerelement strukturiert werden, so dass beispielsweise die Testchemie und das Trägerelement gleichzeitig geschnitten werden können, beispielsweise durch einen der oben beschriebenen Schneideprozesse und/oder einen Schneideprozess in Form eines Stanzprozesses. Verschiedene Ausgestaltungen sind möglich.

Durch das beschriebene Konzept der Abdeckung der mindestens einen Schneidekante lassen sich die oben beschriebenen Vorteile auf hervorragende Weise realisieren. Die Wand der Kammer kann die mindestens eine Öffnung aufweisen, welche im Folgenden auch als Testelementfenster und/oder einfach als Fenster bezeichnet wird und welche einen eingeschränkten Zugang vom Inneren der Kammer zu dem Testfeld bereitstellen kann. Die Wand der Kammer selbst, insbesondere mit der mindestens einen Öffnung, wirkt also in dieser bevorzugten Ausgestaltung als das oben beschriebene Trennelement, welches den Zugang zu dem Testfeld von der Kammer aus ermöglicht und gleichzeitig eine Kontamination des Inneren der Kammer durch die Testchemie, insbesondere durch partikuläre Bestandteile, verhindern kann. So kann das Testfeld, welches als Chemiefeld ausgestaltet sein kann, beispielsweise ganz oder teilweise in das Gehäuse des Magazins integriert werden.

Beispielsweise kann das Testfeld in einer Vertiefung des Magazingehäuses aufgenommen sein, beispielsweise einer von außen zugänglichen Vertiefung, welche durch die mindestens eine Öffnung vom Inneren der Kammer aus zugänglich ist. Beispielsweise kann das Testfeld in der Vertiefung derartig positioniert sein, dass die Schneidekanten des Testfeldes, beispielsweise die Ränder des Testfeldes, vom Magazingehäuse umschlossen werden. Eine Verbindung zwischen dem Inneren der Kammer, beispielsweise eine Verbindung von einem im Inneren der Kammer aufgenommenen Lanzettenelement, hin zu dem Testelement, insbesondere dem Testfeld, kann dann beispielsweise ausschließlich über die genannte Öffnung erfolgen. Die genannte Öffnung ist vorzugsweise klein ausgestaltet, beispielsweise mit einer die von der Kammer aus zugängliche Testfeldfläche begrenzenden Fensterfläche von 0,01 mm² - 5 mm², vorzugsweise 0,05 mm² - 0,5 mm² und besonders bevorzugt 0,25 mm². Auf diese Weise können insbesondere die Schneidekanten des Testelements von dem Magazingehäuse abgedeckt werden, welche beispielsweise für ein Abbröckeln der Testchemie und damit für eine partikuläre Kontamination verantwortlich sein können.

Wie oben dargestellt, kann das Testelement weiterhin insbesondere mindestens eine Lanzette umfassen, vorzugsweise mindestens einen Microsampler, wobei die Lanzette zumindest teilweise in der Kammer aufgenommen sein kann und eingerichtet sein kann, um eine Probe einer Körperflüssigkeit aufzunehmen. Das analytische Magazin kann derart eingerichtet sein, dass die Lanzette in mindestens einer Position die aufgenommene Probe auf das Testfeld übertragen kann. Beispielsweise kann eine Position vorgesehen sein, in welcher die Lanzette und/oder eine Kapillare des Microsamplers derart in die Nähe des Testfeldes gebracht wird, beispielsweise über das oben genannte Testfeldfenster innerhalb der Kammer, dass ein Übertrag möglich ist. Der Übertrag kann auch durch ein oder mehrere zusätzliche Übertragungselemente bewirkt oder zumindest erleichtert werden, beispielsweise durch mindestens einen zusätzlichen Aktor. Mittels dieses Aktors kann beispielsweise auf die Lanzette, beispielsweise den Microsampler, und/oder auf das Testfeld eingewirkt werden, um die Lanzette auf das Testfeld zu drücken und/oder umgekehrt, in der Weise, dass die Probe auf das Testfeld übertragen wird. Dementsprechend kann die Lanzette beispielsweise oberhalb oder unterhalb des genannten mindestens einen Testelementfensters angeordnet sein, beispielsweise mit einer dem Testelementfenster zuweisenden Flachseite der Lanzette.

Der oben genannte Aktor, welcher den Übertrag der Probe von der Lanzette auf das Testfeld bewirkt und/oder erleichtert, kann ganz oder teilweise Bestandteil des analytischen Magazins sein und/oder kann auch ganz oder teilweise Bestandteil eines das analytische Magazin einsetzenden analytischen Systems sein. Im Gehäuse des analytischen Magazins können beispielsweise eine oder mehrere entsprechende Öffnungen vorgesehen sein, welche einen Eingriff des Aktors ermöglichen.

Alternativ oder zusätzlich können in dem analytischen Magazin und/oder dem analytischen System weitere Aktoren vorgesehen sein. Beispielsweise kann das analytische Magazin derart ausgestaltet sein, dass eine Lanzette vorgesehen ist, welche in der Kammer bewegbar gelagert ist. Der mindestens eine Aktor kann derart ausgestaltet sein, dass dieser mittels eines oder mehrerer Koppelelemente, welche auf Seiten der Lanzette und/oder auf Seiten des Aktors vorgesehen sein können, mit der Lanzette zusammenwirken kann. Insbesondere kann das Magazin derart ausgestaltet sein, dass die Lanzette eine Probennahmebewegung durchführt, welche beispielsweise eine Bewegung hin zur Hautoberfläche eines Probanden, einen Stechvorgang bzw. einen Einstichvorgang, einen Sammelvorgang und eine Rückbewegung, weg von der Hautoberfläche des Probanden umfassen kann, und vorzugsweise eine Remagazinierung der Lanzette, beispielsweise in der Kammer, welcher dieser entnommen worden ist. Das mindestens eine Koppelelement kann beispielsweise derart ausgestaltet sein, dass dieses ein Ankoppeln bei Beginn der Probennahmebewegung und ein Abkoppeln beim Ende der Probennahmebewegung bewirkt, vorzugsweise automatisch. Die Probennahmebewegung kann beispielsweise an einer in einer Applikationsposition des analytischen Systems befindlichen Kammer vorgenommen werden. Das analytische Magazin kann beispielsweise derart ausgestaltet sein, dass die Lanzette bei einer Bewegung in der Kammer eine gekrümmte Bahn beschreibt und/oder einer Formänderung unterworfen ist. Durch diese gekrümmte Bahn und/oder die Formänderung der Lanzette kann beispielsweise ein Ankoppeln an den mindestens einen Aktor bewirkt werden. Beispielsweise kann die Lanzette mindestens ein Koppelelement umfassen, welches durch die Krümmung und/oder die Formänderung an ein entsprechendes Koppelelement des Aktors angekoppelt wird, bzw. von diesem abgekoppelt wird. Beispielsweise kann dieses Koppelelement eine Öse und/oder ein Pilotloch der Lanzette umfassen, insbesondere einer Flachlanzette.

Wie oben dargestellt, kann das Magazin insbesondere derart eingerichtet sein, dass die Lanzette in mindestens einer Position die aufgenommene Probe auf das Testfeld übertragen kann. Beispielsweise kann der Aktor eingerichtet sein, um in einer Applikationsposition des analytischen Systems befindlichen Kammer das Testfeld nach der Probennahmebewegung und/oder während der Probennahmebewegung, insbesondere am Ende der Probennahmebewegung, in diese Position zu bringen, welche auch als Übertragungsposition beschrieben werden kann. In dieser Übertragungsposition kann beispielsweise die Lanzette derart relativ zum Testfeld positioniert sein, dass aufgrund eines direkten Kontakts und/oder aufgrund einer räumlichen Nähe eine Übertragung der Probe von der Lanzette auf das Testfeld erfolgen kann. Vorzugsweise ist das analytische Magazin dabei derart eingerichtet, dass die Lanzette in einem Ruhezustand in einer Lagerposition innerhalb der Kammer gelagert ist, welche von der mindestens einen Übertragungsposition verschieden ist. Unter einem Ruhezustand ist dabei insbesondere ein Zustand zu verstehen, in welchem das mindestens eine analytische Hilfsmittel nicht verwendet wird, beispielsweise ein unbenutzter Zustand, in welchem das analytische Hilfsmittel noch nicht verwendet worden ist. Beispielsweise können sich die analytischen Hilfsmittel in unbenutzten Kammern in dieser Lagerposition befinden. Nach der Verwendung der Kammern bzw. der darin aufgenommenen analytischen Hilfsmittel, können dann die Lanzetten in der Übertragungsposition verbleiben oder, alternativ, wieder in die Lagerposition und/oder eine andere Position gebracht werden, beispielsweise wiederum mit Hilfe des mindestens einen Aktors des analytischen Systems.

Die Ausgestaltung, in welcher die Lagerposition von der Übertragungsposition getrennt ist, bewirkt insbesondere, dass beispielsweise ein Kontakt der Lanzette mit dem Testfeld vermieden werden kann. Insbesondere kann die Lagerposition derart ausgestaltet sein, dass in dieser die Lanzette keinen Kontakt mit dem Testfeld aufweist. Auf diese Weise lassen sich Abriebe und dadurch beispielsweise Partikelbildungen weiter reduzieren.

Alternativ oder zusätzlich zu der voranstehend beschriebenen Möglichkeit der Ausgestaltung des Trennelements derart, dass mindestens eine Schneidekante des Testelements durch mindestens eine Wand der Kammer abgedeckt wird, lässt sich das mindestens eine Trennelement auch auf eine andere Weise realisieren. Die im Folgenden beschriebene zweite Realisierungsform kann alternativ oder zusätzlich zu der oben beschriebenen ersten Realisienurgsform ausgestaltet sein. Insbesondere können die oben beschriebenen optionalen Ausgestaltungen auch im Rahmen der im Folgenden beschriebenen zweiten Ausgestaltung des Trennelements eingesetzt werden, auch wenn die übrigen Merkmale des ersten Konzepts der Ausgestaltung des Trennelements nicht realisiert sind. So können beispielsweise die zusätzlichen Merkmale der sich auf das erste Konzept beziehenden Unteransprüche auch, losgelöst von der Realisierung des ersten Konzepts, im Rahmen des im Folgenden beschriebenen zweiten Konzepts realisiert werden.

Wie oben dargestellt, besteht der gemeinsame Grundgedanke darin, dass mindestens ein Trennelement vorgesehen ist, welches einerseits einen zumindest teilweisen Zugang der Probe der Körperflüssigkeit und/oder von Bestandteilen der Probe der Körperflüssigkeit zu dem Testfeld ermöglichen soll, jedoch andererseits in umgekehrter Richtung einen Zugang von Kontaminationen, insbesondere partikulären Kontaminationen, vom Testelement ausgehend hin zum Inneren der Kammer erschweren soll. Erfindungsgemäß wird in dem zweiten Konzept, welches vorliegend auch beansprucht wird, vorgeschlagen, dieses Trennelement derart zu realisieren, dass dieses mindestens eine Membran umfasst oder durch mindestens eine Membran gebildet wird.

Dementsprechend wird ein analytisches Magazin vorgeschlagen, insbesondere gemäß einer oder mehreren der oben beschriebenen optionalen Ausgestaltungen, welches mindestens eine Kammer mit mindestens einem analytischen Hilfsmittel umfasst, das mindestens ein Testelement zum Nachweis mindestens eines Analyten in einer Probe einer Körperflüssigkeit umfasst. Das Testelement umfasst mindestens ein Testfeld mit mindestens einer Testchemie. Für die möglichen Definitionen der Begriffe, sowie mögliche optionale Ausgestaltungen, kann weitgehend auf die obige Beschreibung verwiesen werden. Insbesondere kann das Testelement ganz oder teilweise innerhalb der Kammer angeordnet sein, also wiederum von der Kammer aus zugänglich sein. Insbesondere kann das Testfeld auch wiederum ganz oder teilweise in eine Kammerwand integriert sein, beispielsweise durch Vorsehen mindestens einer Öffnung in Form mindestens eines Testelementfensters, durch welche die Probe der Körperflüssigkeit zu dem mindestens einen Testfeld gelangen kann.

Im Rahmen des zweiten Konzeptes wird vorgeschlagen, zwischen dem Testfeld und einem Innenraum der Kammer zumindest eine Membran anzuordnen. In diesem Fall umfasst das oben genannte Trennelement also eine Membran und/oder wird durch eine derartige Membran gebildet. Die Membran ist ausgestaltet, um zumindest teilweise durchlässig, also permeabel, für die Probe zu sein, insbesondere für Blut oder Blutbestandteile. Die Membran ist jedoch zumindest teilweise undurchlässig für die Testchemie, so dass ein Eindringen von Bestandteilen der Testchemie in die Kammer im Wesentlichen verhindert wird. Unter einer Verhinderung "im Wesentlichen" ist dabei eine Undurchlässigkeit zu verstehen, bei der zumindest feste Chemiebestandteile bis hinunter zu einer Bakteriengröße und größer als 10 Mikrometer zurückgehalten werden.

Unter einer Membran ist dabei ein Element zu verstehen, welches den Durchtritt der Probe und/oder von Bestandteilen der Probe durch dieses Element selbst, zumindest teilweise ermöglicht, welches also zumindest teilweise permeabel für die Probe und/oder von Bestandteilen der Probe ist, insbesondere für Blut und/oder interstitielle Flüssigkeit und/oder andere Körperflüssigkeiten. Die Membran ist ganz oder teilweise als poröses, für die Probe und/oder Bestandteile der Probe durchlässiges Material ausgestaltet. Beispielsweise kann es sich hierbei um ein poröses Kunststoffmaterial handeln. Das poröse Material kann beispielsweise eine Mehrzahl von Poren umfassen, welche beispielsweise den Durchtritt der Probe und/oder von Bestandteilen der Probe ermöglichen, welche jedoch Bestandteile der Testchemie, beispielsweise Partikel der Testchemie, zurückhalten. Beispielsweise kann die Membran ein hydrophiles Material umfassen. Auch andere Ausgestaltungen sind jedoch grundsätzlich möglich. Gleichzeitig ist die Membran derart ausgestaltet, dass diese, zumindest im trockenen Zustand, die Testchemie und/oder Bestandteile der Testchemie, insbesondere möglicherweise toxische und/oder allergische Reaktionen auslösende Elemente der Testchemie, beispielsweise Enzyme, zumindest weitgehend zurückhält. Die Membran ist ausgestaltet, um zumindest im trockenen Zustand für partikuläre Bestandteile der Testchemie mit einer Partikelgröße und/oder einem Durchmesser von mehr als 10 µm zumindest weitgehend undurchlässig zu sein. Es kann sich also um eine semipermeable Membran handeln, welche derartig dimensioniert ist, dass sie im trockenen Zustand impermeabel ist für die Testchemie, insbesondere ein oder mehrere in der Testchemie enthaltene Enzyme, jedoch permeabel ist für die flüssige Probe, insbesondere Blut, so dass ein entsprechender Blutübertrag und/oder ein Übertrag anderer Arten von Proben von Körperflüssigkeit aus dem Inneren der Kammer auf die Testchemie, insbesondere das Testfeld, erfolgen kann.

Das Testfeld, insbesondere eine von der Kammer aus zugängliche Testfeldfläche des Testfeldes, wird durch die Membran vorzugsweise zumindest teilweise, vorzugsweise vollständig, abgedeckt. Die Membran liegt unmittelbar auf dem Testfeld auf, so dass zwischen dem Testfeld und der Membran kein Zwischenraum entsteht oder ein Zwischenraum von 5µm oder weniger. Auf diese Weise kann die Membran bzw. im feuchten Zustand die in der Membran aufgenommene Probe der Körperflüssigkeit selbst als Reservoir der Körperflüssigkeit für die in und/oder an dem Testfeld ablaufende Nachweisreaktion fungieren.

Die Membran kann insbesondere als Folie ausgestaltet sein bzw. mindestens eine Folie umfassen, also ein Element, dessen laterale Ausdehnungen seine Dicke um ein Mehrfaches, vorzugsweise um einen Faktor 10, insbesondere 100 oder mehr übersteigt. Die Membran, insbesondere die semipermeable Membran, kann weiterhin derart ausgestaltet sein, dass diese eine Spreitwirkung in lateraler Richtung, also in einer Richtung senkrecht zur Durchtrittsrichtung der Probe der Körperflüssigkeit, aufweist. Eine derartige Spreitwirkung bedingt, dass sich die Probengeometrie senkrecht zur Durchtrittsrichtung aufweitet, so dass die Probe gleichmäßiger auf das Testfeld bzw. die von der Kammer aus zugängliche Testfeldfläche verteilt wird.

Die Membran ist vorzugsweise, wie oben dargestellt, semipermeabel ausgestaltet und zumindest teilweise durchlässig für Körperflüssigkeiten, aber zumindest teilweise undurchlässig für Bestandteile der Testchemie, insbesondere partikuläre Bestandteile. Allgemein sind die Anforderungen an die Membran derart, dass diese insbesondere eine Rückhaltewirkung für partikuläre Bestandteile der Testchemie aufweisen kann. Grundsätzlich kann die Membran hierzu beispielsweise eine mittlere Porengröße, insbesondere angebbar als so genannter d₅₀-Wert, zwischen 1 µm und 10 µm, vorzugsweise zwischen 2 µm und 5 µm aufweisen. Insbesondere kann die Membran ein Porennetzwerk umfassen, wobei die mittleren Porengrößen, welche auf übliche Weise bestimmt werden können, der genannten Bedingung genügen können. Unter einer Porengröße kann dabei beispielsweise ein mittlerer Durchmesser und/oder mittlerer Äquivalentdurchmesser aller einzelnen Poren innerhalb der Membran angesehen werden. Bevorzugt sind symmetrische Membranen mit durchgehend gleicher Porenverteilung zwischen den beiden Seiten und/oder über die gesamte Dicke der Membran.

Poren mit einer Porengröße von weniger 10 µm oder kleiner sind insbesondere geeignet, um partikuläre Bestandteile am Eindringen in das Innere der Kammern zu hindern. Weiterhin sind derartige Poren noch geeignet, um typische mikrobiologische Verunreinigungen, insbesondere Bakterien, am Eindringen ins Innere der Kammern durch die Membran hindurch zu hindern. Auf diese Weise kann beispielsweise ein Sterilschutz aufrechterhalten werden. Andererseits garantiert die bevorzugte Untergrenze, nach welcher die Porengröße minimal 1µm oder mehr beträgt, dass die Probe, insbesondere eine wässrige Probe, wie beispielsweise Blut und/oder interstitielle Flüssigkeit, zumindest teilweise durch die Membran hindurch treten kann. Bei Porengrößen unterhalb von 1µm ist ein Zugang der Probe zum Testfeld vom Inneren der Kammer aus zumindest stark erschwert.

Die Membran ist, wie oben dargestellt, vorzugsweise als Folienmembran ausgestaltet. Die Membran kann insbesondere eine Dicke, also eine Ausdehnung parallel zur Durchtrittsrichtung der Probe der Körperflüssigkeit hin zu dem Testfeld, aufweisen, welche vorzugsweise zwischen 30 µm und 150 µm liegt, insbesondere zwischen 50 µm und 100 µm. Diese bevorzugte Dicke wirkt sich nicht nur auf die oben beschriebene Rückhaltewirkung für partikuläre Verunreinigungen und/oder mikrobielle Verunreinigungen, bei gleichzeitiger Durchlässigkeit für die Probe, aus. So kann die oben genannte bevorzugte Dicke der Membran auch die oben beschriebene Bereitstellung eines Flüssigreservoirs der Probe der Körperflüssigkeit oberhalb des Testfeldes bewirken. So hat es sich insbesondere bei bekannten Testchemien, insbesondere zum Nachweis von Blutglukose gezeigt, dass eine Flüssigkeitssäule oberhalb des Testfeldes von mindestens 30 µm, vorzugsweise von mindestens 50 µm, wünschenswert ist. Kleinere Flüssigkeitssäulen, beispielsweise kleinere Blutsäulen, bewirken, dass aufgrund von Diffusionseffekten die Nachweisreaktion abhängig werden kann von der tatsächlichen Höhe der Blutsäule. Bei Dicken von 30 µm oder mehr, vorzugsweise 50 µm oder mehr, wird hingegen in den meisten Fällen die Reaktion, beispielsweise eine Farbreaktion, zum Nachweis des mindestens einen Analyten weitgehend unabhängig von der tatsächlichen Höhe der Flüssigkeitssäule. Andererseits wirkt sich jedoch die zunehmende Dicke der Membran negativ auf die Durchlässigkeit der Membran für die flüssige Probe und auf das nötige Probenvolumen aus, so dass die genannten Obergrenzen der Dicke von maximal 150 µm, insbesondere maximal 100 µm, zu bevorzugen sind.

Für die Membran können grundsätzlich beliebige organische und/oder anorganische Materialien eingesetzt werden, welche die oben genannten Eigenschaften erfüllen. Diese können in Form einer oder mehrerer geschlossenen Schichten aufgebracht werden und/oder auch in Form strukturierter Materialien. Bevorzugt sind geschlossene, poröse Materialien. Die Membran kann insbesondere hydrophile Eigenschaften aufweisen. Insbesondere kann die Membran ein Kunststoffmaterial, vorzugsweise ein hydrophiles Kunststoffmaterial umfassen und/oder vollständig aus einem derartigen Kunststoffmaterial hergestellt sein. Insbesondere soll es sich bei dem Membranmaterial um ein Material handeln, welches beim Sterilisieren des analytischen Magazins, beispielsweise mittels Elektronenstrahlsterilisation und/oder Gamma-Sterilisation, seine Eigenschaften im Wesentlichen nicht verändert.

Besonders bevorzugt ist die Verwendung eines oder mehrerer der folgenden Materialien: eine Zellulose und/oder ein Zellulosederivat, insbesondere eine Nitrozellulose; ein Polysulfon; ein Nylon; ein Polyvinylidenfluorid; eine regenerierte Zellulose; ein hydrophiles Polyurethan. Insbesondere lassen sich Materialien einsetzen, welche typischerweise in Filtern, beispielsweise Wasserfiltern, zum Einsatz kommen. Alternativ oder zusätzlich zu Materialien, welche von sich aus hydrophile Eigenschaften aufweisen, eignen sich auch weniger hydrophile Materialien, welche nachträglich hydrophilisiert wurden, beispielsweise mit einem Netzmittel. Geeignete Netzmittel sind dem Fachmann bekannt.

Die Membran kann beispielsweise mittels eines oder mehrerer Membrankörper in das analytische Magazin eingebracht sein. Beispielsweise kann die Membran auch gleichzeitig für mehrere Kammern bereitgestellt werden. So kann beispielsweise ein Membrankörper vorgesehen sein, mit einzelnen Membranen, welche jeweils einer Kammer zugeordnet sind. Auf diese Weise lässt sich die Herstellung der Membran stark vereinfachen.

Die Membran kann beispielsweise mindestens eine Strukturierung aufweisen, mit mindestens einer Durchtrittsfläche, welche die oben genannten Eigenschaften hinsichtlich der Permeabilität für die Probe und der Impermeabilität für die Testchemie aufweist und welche für die Probe der Körperflüssigkeit zumindest teilweise durchlässig ist, und mindestens einer undurchlässigen Fläche, welche für die Probe der Körperflüssigkeit zumindest teilweise undurchlässig ist. Die Durchtrittsfläche bildet also die eigentliche Membran mit den oben genannten Eigenschaften, also das genannte, mindestens eine Trennelement oder einen Teil desselben. Diese Durchtrittsfläche kann beispielsweise ein Bereich sein, in dem innerhalb der Kammern ein Blutübertrag von der Lanzette auf das Testfeld stattfinden kann.

Um eine Membran in der genannten Weise zu strukturieren, können verschiedene Verfahren eingesetzt werden. So kann beispielsweise die Strukturierung der Membran in Durchtrittsflächen und undurchlässige Flächen durch eine entsprechende Beschichtung der Membran erfolgen, bei welcher beispielsweise die undurchlässigen Flächen mit einer undurchlässigen Beschichtung versehen werden. Alternativ oder zusätzlich kann die Strukturierung jedoch auch beispielsweise durch thermische Verfahren erfolgen, beispielsweise indem in den undurchlässigen Flächen der Membranporen und/oder andere Öffnungen der Membran gezielt geschlossen werden, beispielsweise durch Einbringen von Verschlussmaterialien wie beispielsweise Klebern und/oder durch Verschmelzen des Membranmaterials. Dies kann beispielsweise durch eine Laserstrukturierung erfolgen, bei welcher beispielsweise in den undurchlässigen Flächen das Material der Membran zumindest teilweise verschmolzen wird, so dass die undurchlässige Eigenschaft hergestellt wird.

Sind mehrere Kammern vorgesehen, so kann beispielsweise pro Kammer jeweils mindestens eine Durchtrittsfläche vorgesehen sein. Diese mindestens eine Durchtrittsfläche kann beispielsweise den oben beschriebenen Öffnungen in der Kammerwand entsprechen und/oder diese Öffnungen vollständig abdecken bzw. in diese Öffnungen eingebracht sein. Beispielsweise können die Durchtrittsflächen von ihrer Geometrie her entsprechend den genannten Öffnungen, insbesondere den Testelementfenstern, angepasst sein bzw. beispielsweise geringfügig größer als diese Öffnungen ausgestaltet sein und entsprechend dieser Öffnungen angeordnet sein und/oder beispielsweise diese Öffnungen vollständig oder teilweise bedecken.

Die Membran kann auch für mehrere Kammern gleichzeitig ausgestaltet sein. Beispielsweise kann die Membran für alle Kammern gemeinsam ausgebildet werden und für die mehreren Kammern, insbesondere für alle Kammern, jeweils mindestens eine Durchtrittsfläche bereitstellen. Beispielsweise kann die Membran einen gemeinsamen Membrankörper für alle Kammern umfassen, mit jeweils mindestens einer Durchtrittsfläche für jede Kammer. Der gemeinsame Membrankörper kann beispielsweise einen Membranring umfassen und kann beispielsweise in eine Vertiefung des Gehäuses des Magazins eingebracht sein oder auf andere Weise mit dem Gehäuse des Magazins verbunden sein und/oder in dieses Gehäuse integriert sein.

Das analytische Magazin kann, beispielsweise, wie oben aufgeführt, ein Gehäuse umfassen. Dieses Gehäuse kann auch aus mehreren Gehäuseteilen bestehen. Das Testelement kann ganz oder teilweise in das Gehäuse integriert sein. Das Gehäuse kann mindestens eine der mindestens einen Kammer zuweisende Öffnung aufweisen, beispielsweise die oben beschriebenen Testfeldfenster, vorzugsweise mindestens eine Öffnung pro Kammer. Die Membran kann beispielsweise von außen auf die Öffnung aufgebracht sein und durch die Öffnung, welche oben als Fenster bezeichnet wurde, von der Kammer aus zugänglich sein. Das Testfeld ist vorzugsweise von außen auf die Membran aufgebracht, so dass beispielsweise, von der Kammer bzw. dem Inneren der Kammer aus betrachtet, durch die Öffnung zunächst die Membran angeordnet ist, gefolgt von dem mindestens einen Testfeld.

Die Membran kann insbesondere mit dem Gehäuse verbunden sein. Diese Verbindung kann grundsätzlich durch beliebige Verbindungsarten erfolgen, welche auch kombiniert werden können. Besonders bevorzugt sind formschlüssige Verbindungen. Besonders bevorzugt umfasst die mindestens eine Verbindung eine Schweißverbindung und/oder ein Warmverstemmen. Besonders bevorzugt sind hierbei aufgrund der geringen Erschütterung beim Schweißen Laser-Schweißverbindungen. Alternativ oder zusätzlich können jedoch auch thermische Schweißverbindungen und/oder Ultraschall-Schweißverbindungen und/oder andere Arten von Schweißverbindungen eingesetzt werden. Alternativ oder zusätzlich zu Schweißverbindungen können auch beispielsweise Klebeverbindungen verwendet werden, also Verbindungen, bei welchen die Membran vollständig oder teilweise auf das Gehäuse aufgeklebt wird. Beispielsweise kann dies derart erfolgen, dass jeweils mindestens eine Durchtrittsfläche vor mindestens einer Öffnung des Gehäuses positioniert wird.

Zum Aufnehmen der mindestens einen Membran können das Gehäuse oder mindestens ein Gehäuseteil des Gehäuses Elemente umfassen, welche die Verbindung zwischen der Membran und dem Gehäuse und/oder einer Positionierung der Membran relativ zu dem Gehäuse erleichtern. Beispielsweise können entsprechende Aufnahmeelemente, wie Vertiefungen, Nuten, Haken, Ösen, Vorsprünge oder ähnliches oder Kombinationen der genannten und/oder anderer Elemente vorgesehen sein. Beispielsweise kann, wie oben ausgeführt, die Membran in Form eines Membranrings ausgestaltet sein, welcher beispielsweise in eine entsprechende Ringnut in dem Gehäuse eingebracht werden kann.

Wie oben dargestellt, kann auch das mindestens eine Testfeld ganz oder teilweise in das Gehäuse integriert sein. So kann das Testfeld beispielsweise mit dem Gehäuse und/oder mindestens einem Gehäuseteil verbunden werden und somit selbst zu einem Bestandteil des Gehäuses werden. Die Verbindung des Testfeldes mit dem Gehäuse kann grundsätzlich wiederum auf verschiedene Weisen erfolgen. Besonders bevorzugt sind allgemein kraftschlüssige und/oder formschlüssige Verbindungen, insbesondere Clipverbindungen.

Beispielsweise kann ein Testfeld durch eine derartige Verbindung von außen auf das Gehäuse aufgebracht werden, beispielsweise aufgeclipst werden. Dieses Aufbringen erfolgt vorzugsweise derart, dass die oben beschriebenen optionalen Öffnungen, optional mit der eingebrachten und/oder darüber liegenden Membran, vollständig oder teilweise abgedeckt werden.

Die kraftschlüssige und/oder formschlüssige Verbindung kann beispielsweise durch ein oder mehrere Verbindungselemente erleichtert werden, welche an dem Gehäuse und/oder an mindestens einem Gehäuseteil vorgesehen sein können. So können beispielsweise entsprechende Rast-Verbindungselemente an dem Gehäuse vorgesehen sein und/oder andere Arten von Verbindungselementen, beispielsweise Nuten, Vorsprünge, Haken oder ähnliches, oder Kombinationen der genannten und/oder anderer Verbindungselemente. Beispielsweise kann das Testfeld in Form eines Chemierings und/oder einer Chemiescheibe von außen auf das Gehäuse aufgeclipst werden.

Ein wesentlicher Vorteil des analytischen Magazins gemäß der Erfindung besteht darin, dass das Verbinden des Testfeldes mit dem Gehäuse auch zeitlich unabhängig und/oder prozessual unabhängig von dem Verbinden der mindestens einen Membran mit dem Gehäuse erfolgen kann. Auf diese Weise wird es ermöglicht, sterile Halbfertigelemente des analytischen Magazins zu erzeugen, welche gegebenenfalls sterilisiert und/oder zwischengelagert werden können, um anschließend die Testfelder aufzubringen. So kann beispielsweise ein Herstellungsverfahren für das analytische Magazin verwendet werden, bei welchem zunächst die Membran mit dem Gehäuse und/oder mindestens einem Gehäuseteil des analytischen Magazins verbunden wird. Dies erfolgt vorzugsweise derart, dass die mindestens eine Kammer durch die mindestens eine Membran derart abgedichtet wird, dass, zumindest durch die mindestens eine Membran, vorzugsweise keine partikulären Verunreinigungen und/oder mikrobiellen Verunreinigungen, insbesondere keine Bakterien, ins Innere der Kammer mehr eindringen können.

Zuvor oder anschließend können weitere analytische Hilfselemente und/oder Teil-Hilfselemente, wie beispielsweise eine oder mehrere Lanzetten, in die mindestens eine Kammer eingebracht werden. Auf diese Weise können die Lanzetten durch die Membran geschützt werden, insbesondere steril gelagert werden. Nach der Verbindung der mindestens einen Membran mit dem Gehäuse kann dann optional eine Sterilisierung des teilweise fertig gestellten Magazins erfolgen, beispielsweise eine Sterilisierung durch Gammastrahlung und/oder eine Sterilisierung durch Elektronenstrahlung. Die derartigen teilweise fertig gestellten Magazine können dann beispielsweise vorübergehend gelagert werden und gegebenenfalls eine gemeinsame Plattform für verschiedenste Arten von analytischen Magazinen, beispielsweise mit unterschiedlichen Testchemien bereitstellen. In einem weiteren Verfahrensschritt, welcher auch zeitlich nachgelagert werden kann, kann dann das mindestens eine Testfeld mit dem Gehäuse verbunden werden, beispielsweise durch eine oder mehrere der oben beschriebenen Verbindungsarten. Dabei kann grundsätzlich, entsprechend dem genannten Plattformgedanken, eine beliebige Auswahl der Testchemie erfolgen. So kann beispielsweise eine Charge von analytischen Magazinen erst in diesem Verfahrensschritt definiert werden, entsprechend beispielsweise der aufgebrachten mindestens einen Testchemie.

Neben dem analytischen Magazin in einer oder mehreren der oben beschriebenen Ausführungsformen wird weiterhin ein Verfahren zur Herstellung eines analytischen Magazins offenbart, jedoch vorliegend nicht separat beansprucht. Das Verfahren kann insbesondere zur Herstellung eines analytischen Magazins nach einer oder mehreren der oben beschriebenen Ausführungsformen eingesetzt werden, so dass für mögliche Ausgestaltungen des Verfahrens auf die oben beschriebenen möglichen Ausgestaltungen des analytischen Magazins verwiesen werden kann. Das Verfahren kann entsprechend durch einen oder mehrere Verfahrensschritte ergänzt werden, in welchen die bevorzugten Optionen realisiert werden. Das analytische Magazin umfasst mindestens eine Kammer mit mindestens einem analytischen Hilfsmittel. Das analytische Hilfsmittel umfasst mindestens ein Testelement zum Nachweis mindestens eines Analyten in einer Probe einer Körperflüssigkeit. Das Testelement umfasst mindestens ein Testfeld mit mindestens einer Testchemie. Bei dem Verfahren wird mindestens ein Lanzettenelement, beispielsweise ein Microsampler, in die Kammer eingebracht.

Das Verfahren weist weiterhin die folgenden Verfahrensschritte auf, welche vorzugsweise, jedoch nicht notwendigerweise, in der dargestellten Reihenfolge durchgeführt werden. Es können auch einzelne oder mehrere Verfahrensschritte zeitlich überlappend und/oder zeitlich parallel und/oder wiederholt durchgeführt werden. Weiterhin kann das Verfahren zusätzliche, im Folgenden nicht aufgeführte Verfahrensschritte umfassen. Das Verfahren umfasst folgende Schritte:
- mindestens ein Gehäuseteil eines Gehäuses des analytischen Magazins wird hergestellt;
- das Testfeld wird hergestellt; und
- das Testfeld und/oder das Testelement wird derart mit dem Gehäuseteil verbunden, dass das Testfeld zumindest teilweise von der Kammer aus für eine Probenaufgabe zugänglich ist, wobei mindestens eine Wand der Kammer das Testfeld zumindest teilweise überdeckt und dadurch eine von der Kammer aus zugängliche Testfeldfläche zumindest teilweise begrenzt.

Das Verfahren kann insbesondere derart durchgeführt werden, dass das Testelement und/oder das Testfeld unter Verwendung eines Schneideverfahrens hergestellt wird. Die Wand der Kammer kann insbesondere, wie oben beschrieben, mindestens eine Schneidekante des Testfeldes überdecken.

Als weiterer Verfahrensschritt, insbesondere vor dem Verbinden des Testfeldes und/oder Testelements mit dem Gehäuseteil, kann in einem weiteren Verfahrensschritt beispielsweise mindestens eine Membran eingebracht werden, beispielsweise indem diese ebenfalls mit dem Gehäuseteil verbunden wird. Für weitere Ausgestaltungen, insbesondere hinsichtlich der Ausgestaltung und/oder Anordnung der Membran, kann beispielsweise auf die obige Beschreibung verwiesen werden.

Beispielsweise kann zunächst die Membran mit dem Gehäuse und/oder dem mindestens einen Gehäuseteil verbunden werden, so dass ein Halbfertigteil entstehen kann. Dieses Halbfertigteil kann beispielsweise sämtliche Teile des analytischen Magazins bis auf das Testelement und/oder das Testfeld umfassen. Das Halbfertigteil kann beispielsweise sterilisiert und/oder zwischengelagert werden. Zu einem späteren Zeitpunkt kann das Halbfertigteil dann durch den beschriebenen Verfahrensschritt des Anfügens des Testelements und/oder des Testfeldes zu dem analytischen Magazin ergänzt werden.

Das Verbinden des Testfeldes und/oder des Testelements mit dem Gehäuseteil kann zu einem Zeitpunkt erfolgen, zu welchem das Gehäuse bereits nahezu (das heißt insbesondere bis auf das Testelement) fertig gestellt ist oder zu einem anderen Zeitpunkt. So können beispielsweise mehrere Gehäuseteile vorgesehen sein, welche zusammengefügt werden. Das Verbinden des Testelements und/oder Testfelds mit dem mindestens einen Gehäuseteil kann in diesem Fall vor, während oder nach dem Zusammenfügen der Gehäuseteile erfolgen. Insbesondere muss die mindestens eine Kammer zu dem Zeitpunkt, zu welchem das Verbinden des Testelements und/oder Testfelds mit dem Gehäuseteil erfolgt, noch notwendigerweise nicht vollständig fertig gestellt sein, sondern es kann beispielsweise auch ein Verfahren verwendet werden, bei welchem die Kammer erst zu einem späteren Zeitpunkt, beispielsweise nach Einbringung weiterer Gehäuseteile, fertig gestellt wird. Weiterhin muss die Bedeckung des Testfeldes durch die Kammerwand, wodurch eine von der Kammer aus zugängliche Testfeldfläche begrenzt wird, nicht notwendigerweise durch das Gehäuseteil erfolgen, mit welchem das Testelement und/oder das Testfeld verbunden ist, sondern die Wand kann, zusätzlich oder alternativ, auch zumindest teilweise von einem oder mehreren anderen Gehäuseteilen gebildet werden.

Das analytische Magazin gemäß einer oder mehreren der oben beschriebenen Ausführungsformen und das vorgeschlagene Verfahren weisen gegenüber bekannten analytischen Magazinen, insbesondere integrierten analytischen Magazinen mit mindestens einer Lanzette und mindestens einem Testelement, sowie gegenüber bekannten Herstellungsverfahren zahlreiche Vorteile auf. So kann eine Kontamination von Lanzetten in den Kammern der analytischen Magazine, insbesondere durch mikrobielle Verunreinigungen und/oder partikuläre Verunreinigungen, wirkungsvoll verhindert werden. Gleichzeitig lässt sich jedoch eine hohe Systemintegration realisieren, da innerhalb der Kammern Testelemente und Lanzetten gemeinsam aufgenommen werden können.

Weiterhin lassen sich die analytischen Magazine technisch vergleichsweise einfach realisieren, da die genannten Trennelemente in wenigen Produktionsschritten und unter Verwendung weniger Einzelteile für die Herstellung der analytischen Magazine bereitgestellt werden können. Insbesondere lassen sich Magazine mit einer trockenen Montage der mindestens einen Membran über oder unter einem separat ausgebildeten Testfeld auf einfache Weise realisieren.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt, gleiche Bezugsziffern in den einzelnen Figuren bezeichnen dabei gleiche oder funktionsgleiche bzw. ihrer Funktion einander entsprechende Elemente.

Im Einzelnen zeigt:
- Figur 1: eine Explosionsdarstellung eines Ausführungsbeispiels eines analytischen Magazins;
- Figuren 2A bis 2E: verschiedene Einzelteile des analytischen Magazins gemäß Figur 1;
- Figur 3: eine perspektivische Schnittdarstellung durch eine Kammer des analytischen Magazins gemäß Figur 1; und
- Figuren 4A und 4B: ein weiteres Ausführungsbeispiel einer erfindungsgemäßen Kammer eines analytischen Magazins mit einer Lanzette in verschiedenen Positionen.

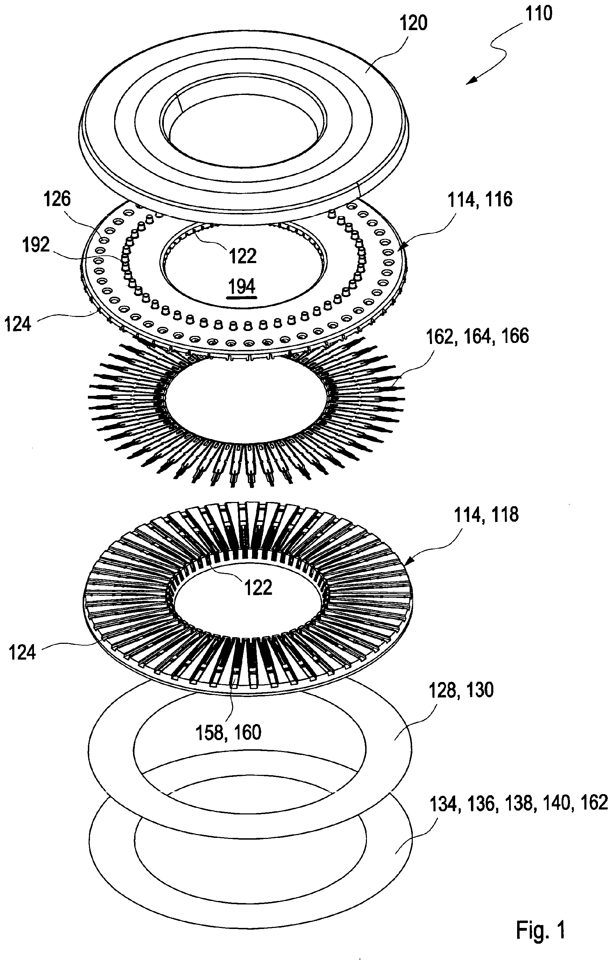

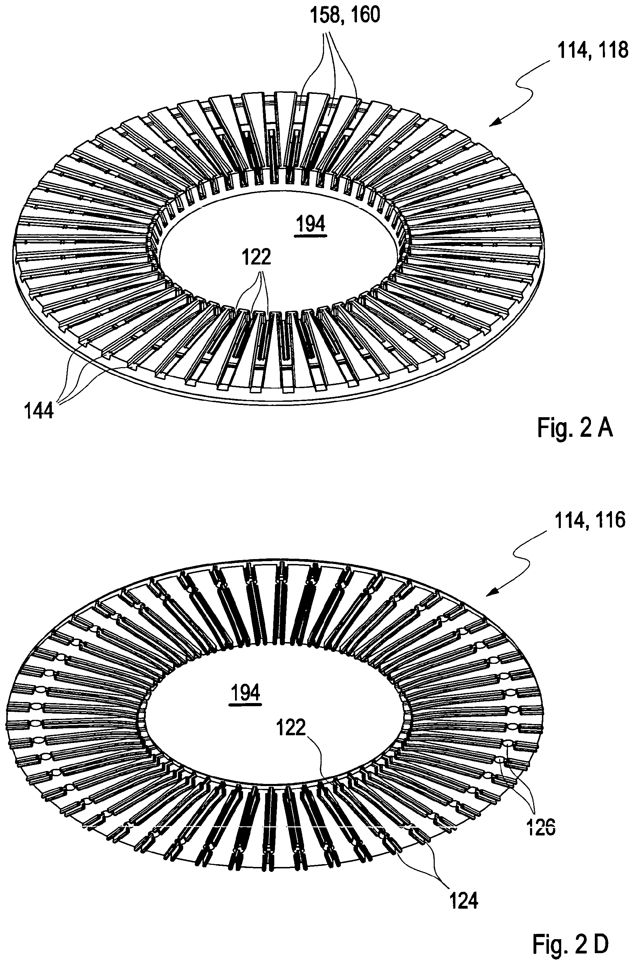

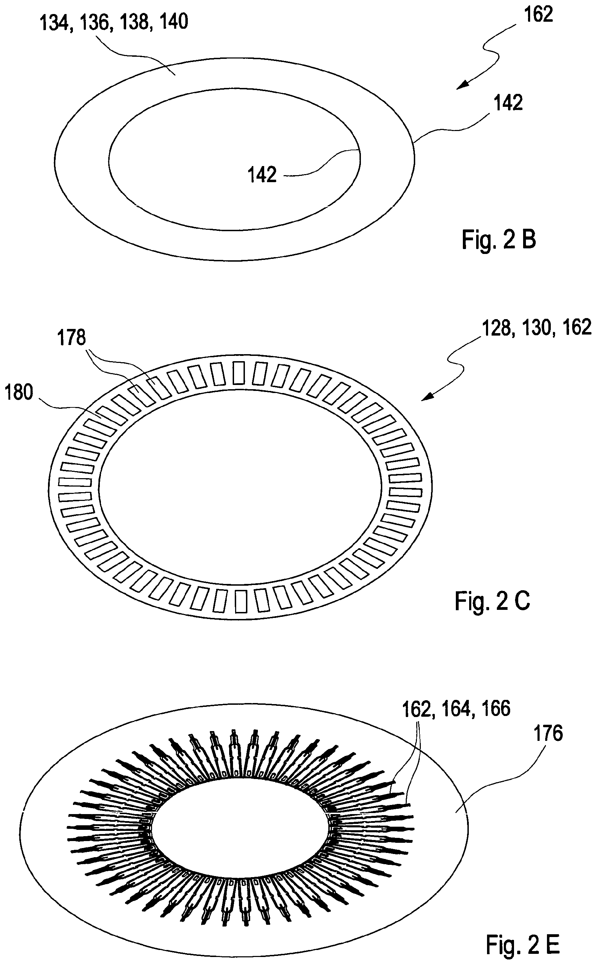

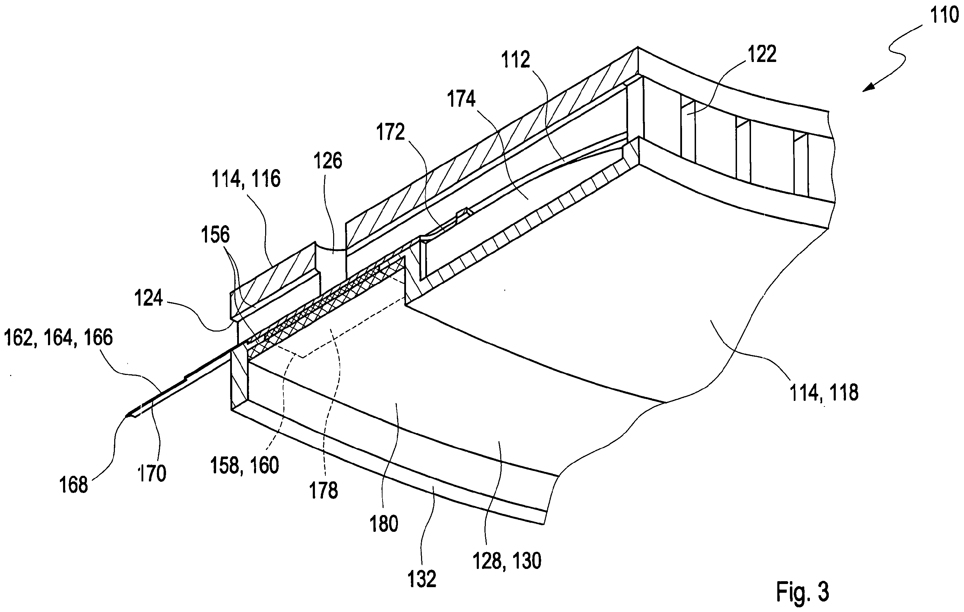

In den Figuren 1 bis 3 ist ein erstes Ausführungsbeispiel eines erfindungsgemäßen analytischen Magazins 110 dargestellt. Diese Figuren werden im Folgenden gemeinsam beschrieben. Das analytische Magazin 110 kann beispielsweise in einem in den Figuren nicht dargestellten analytischen System eingesetzt werden. In Figur 1 ist das analytische Magazin 110 in einer Explosionsdarstellung gezeigt, wohingegen in den Figuren 2A bis 2E einige Bauteile des analytischen Magazins 110 gezeigt sind. Figur 3 zeigt eine perspektivische Schnittdarstellung durch eine Kammer 112 des analytischen Magazins 110.

Das analytische Magazin 110 umfasst ein in Figur 1 mit der Bezugsziffer 114 bezeichnetes Gehäuse, welches mehrteilig ausgebildet ist. Dieses Gehäuse 114 umfasst ein erstes Gehäuseteil in Form einer Oberschale 116 und ein zweites Gehäuseteil in Form einer Unterschale 118. Die Unterschale 118 ist in perspektivischer Darstellung schräg von oben nochmals in Figur 2A gezeigt, wohingegen die Oberschale 116 nochmals in perspektivischer Darstellung schräg von unten in Figur 2D gezeigt ist.

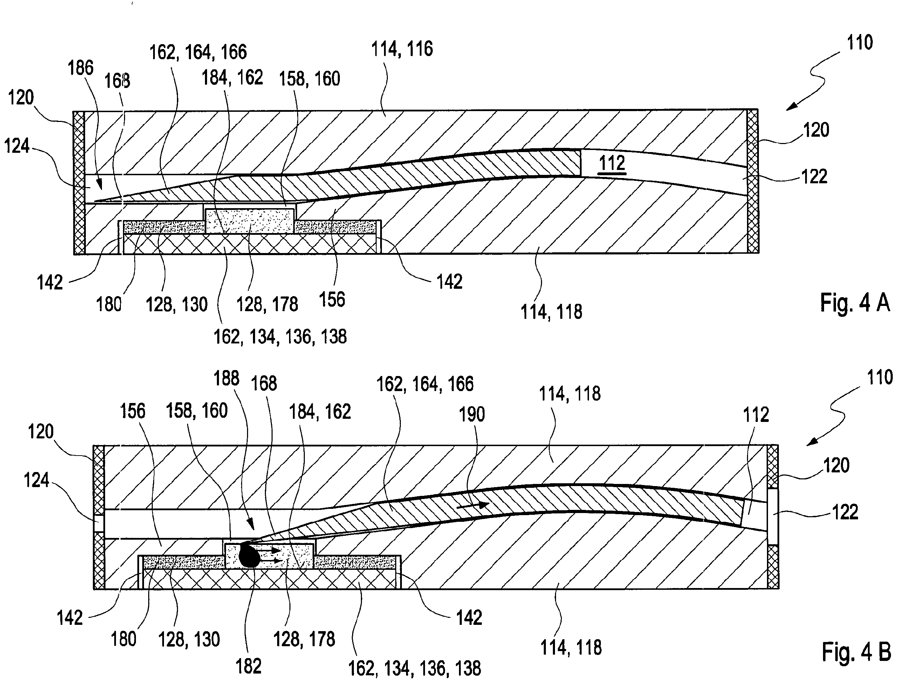

Weiterhin umfasst das analytische Magazin 110 eine oder mehrere optionale Versiegelungen 120, welche lediglich in der perspektivischen Darstellung gemäß Figur 1 erkennbar sind und welche beispielsweise eingesetzt werden können, um Innenöffnungen 122 und/oder Außenöffnungen 124 und/oder Aktoröffnungen 126 (siehe insbesondere die Figuren 4A, 4B und 3) zu versiegeln. Die Versiegelung 120 kann beispielsweise eine Kunststofffolie und/oder eine metallische Folie umfassen, welche beispielsweise in einem Tiefziehverfahren geformt werden kann und auf die Öffnungen 122, 124 und/oder 126 aufgebracht, beispielsweise auflaminiert oder aufgebügelt, werden kann.

Weiterhin umfasst das analytische Magazin 110 unterhalb der Unterschale 118 eine Membran 128 in Form eines Membranrings 130, welche in das Gehäuse 114 integriert sein kann und somit Bestandteil des Gehäuses 114 bilden kann. Dieser Membranring 130 kann in eine Ringnut 132 auf der Unterseite der Unterschale 118 eingebracht sein und optional mit der Unterschale 118 beispielsweise durch einen Schweißprozess verbunden werden. Der Membranring 130 ist in Figur 2C in einer Draufsicht gezeigt und wird nachfolgend noch näher beschrieben.

Weiterhin umfasst das analytische Magazin 110 Testelemente 134, welche in diesem Ausführungsbeispiel als für alle Kammern 112 gemeinsames Testfeld 136 in Form eines Testchemierings 138 dargestellt sind. Dieser Testchemiering 138 kann beispielsweise eine Testchemie 140 auf seiner der Unterschale 118 zuweisenden Seite umfassen, insbesondere eine im Wesentlichen feuchtigkeitsstabile Testchemie, beispielsweise gemäß der in WU 2007/012494 A1 dargestellten Art.

Auch andere Arten von Testchemien sind grundsätzlich denkbar. Der Testchemiering 134 kann beispielsweise weiterhin eine oder mehrere Trägerelemente umfassen, beispielsweise ebenfalls in Form von Ringen, so dass die eigentliche Testchemie 140 und der Träger beispielsweise deckungsgleich als Ring ausgebildet sein können. Der Testchemiering 138 weist an seinem Inneren Umfang und an seinem äußeren Umfang jeweils Schneidekanten 142 auf. Der Testchemiering, welcher in Figur 3 nicht dargestellt ist, kann beispielsweise ebenfalls in die Ringnut 132 auf der Unterseite der Unterschale 118 eingebracht werden. Der Testchemiering 138 kann beispielsweise mit der Unterschale 118 durch ein oder mehrere Verbindungselemente verbunden werden, beispielsweise durch Clipverbindungen. Diese Verbindungselemente, welche auch an der Unterschale 118 ausgebildet sein können, sind in Figur 3 ebenfalls nicht gezeigt.

Die Kammern 112 werden durch Vertiefungen 144 in der Oberschale 116 bzw. der Unterschale 118 gebildet, welche jeweils miteinander korrespondieren und bei zusammengefügter Oberschale 116 und Unterschale 118 Wände 156 der Kammern 112 bilden.

In der Unterschale 118 sind weiterhin, der Ringnut 132 zuweisend, Öffnungen 158 ausgebildet, welche als Testelementfenster 160 wirken. Diese Öffnungen 158 sind in Figur 3 durch eine gestrichelte Linie in einem Fall angedeutet, rechteckig ausgestaltet, wobei im dargestellten Beispiel jeweils eine Öffnung 158 einer Kammer 112 zugeordnet ist. Die Öffnungen 158 sind, wie aus Figur 3 ebenfalls hervorgeht, durch den Membranring 130 vollständig überdeckt. Durch die Öffnungen 158 sind somit vom Inneren der Kammern 112 aus lediglich eingeschränkten Testfeldflächen des gesamten Testfeldes 136 zugänglich. Diese Testfeldflächen werden somit im Wesentlichen durch die Öffnung 158 definiert. Wird der Testchemiering 138 in die Ringnut 132 eingelegt, so überdecken die Kanten der Öffnungen 158 und somit die Wände 156 der Kammern 112 die Schneidekanten 142 des Testchemierings 138, so dass diese vom Inneren der Kammern 112 aus nicht zugänglich sind.

Das Testfeld 136 bzw. die jeweils von den Kammern 112 aus zugänglichen Testfeldflächen des Testfeldes 136 sind Bestandteil von in den Kammern 112 angeordneten analytischen Hilfsmitteln 162. Weiterhin umfassen diese analytischen Hilfsmittel 162 Lanzetten 164 in Form von so genannten Microsamplern 166. Diese Microsampler 166 umfassen neben einer Lanzettenspitze 168 jeweils von der Lanzettenspitze 168 weg verlaufende Kapillarspalte 170, welche insbesondere in der perspektivischen Darstellung gemäß Figur 3 erkennbar sind und welche eingerichtet sind, um bei einem Einstich in eine Haut eines Probanden eine Probe an Körperflüssigkeit, insbesondere Blut, aufzunehmen.

Die Microsampler 166 umfassen weiterhin an ihrem hinteren, den Innenöffnungen 122 zuweisenden Ende Koppelelemente 172, welche im dargestellten Ausführungsbeispiel in Form von Ösen und/oder Pilotlöchern ausgestaltet sind. Ein in den Figuren nicht dargestellter Aktor eines analytischen Systems kann durch die Innenöffnungen 122 und eine Aktornut 174, welche Bestandteil der Vertiefungen 144 in der Unterschale 118 und/oder der Oberschale 116 sein kann, in die Kammern 112 eindringen, wobei beispielsweise die Versiegelung 120 an den Innenöffnungen 122 perforiert werden kann. Der Aktor kann ebenfalls Koppelelemente umfassen, beispielsweise Haken, welche in die Koppelelemente 172 der Lanzetten 164 eingreifen können. Wie aus Figur 3 hervorgeht, sind die Lanzetten 164 in den Kammern 112 beweglich gelagert, auf einer gekrümmten Bahn. Sind die Lanzetten 164 mit ihrem hinteren Ende in der Nähe der Innenöffnungen 122 angeordnet, so wird das Koppelelement 172 jeder Lanzette vom Haken der Aktoren weggebogen und entkoppelt. Werden die Lanzetten 164 hingegen nach vorne, zur Außenöffnung 124 hin verschoben, so wird aufgrund der Formung des Hohlraums der Kammern 112 die Lanzette gestreckt, die Koppelelemente 172 bewegen sich nach oben und rasten in den entsprechenden Haken der Aktoren ein.

Ein Test mittels eines analytischen Hilfsmittels 162, beispielsweise unter Verwendung eines entsprechenden analytischen Systems, kann insbesondere auf die folgende Weise durchgeführt werden. Zunächst dringt eine Aktorstange durch eine Innenöffnung 122 in den Innenraum einer Kammer 112 in eine Applikationsposition des Systems ein und koppelt an das Koppelelement 172 der Lanzette 164 an. Die Lanzette 164 wird nach vorne getrieben, perforiert die Versiegelung 120 an der Außenöffnung 124 und führt eine Probennahmebewegung durch. Dabei wird die Lanzettenspitze 168 in eine Haut eines Probanden getrieben, erzeugt eine Öffnung in der Haut des Probanden und stellt somit eine Probe einer Körperflüssigkeit, beispielsweise einer Blutprobe und/oder ein Probe interstitieller Flüssigkeit bereit. Diese Probe wird durch die Lanzette 164, insbesondere den Kapillarspalt 170 des Microsamplers 166 aufgenommen. Anschließend kann, ebenfalls Bestandteil der Probennahmebewegung, eine Rückwärtsbewegung der Lanzette 164 in die Kammer 112 hineinfolgen, bei welcher die Lanzette 164 auch remagaziniert werden kann. Anschließend kann ein Aktorbolzen des Systems von der Oberschale 116 her durch die korrespondierende Aktoröffnung 126 in das Innere der Kammer 112 eindringen und den Microsampler 166 auf das Testfeld 136 drücken, so dass die Probe auf das Testfeld 136 übertragen wird. Alternativ oder zusätzlich kann auch ein Druck auf das Testfeld 136 ausgeübt werden, um dieses der Lanzette 164 anzunähern und so den Übertrag zu gewährleisten. Auch andere Übertragsmechanismen sind denkbar, wie unten anhand der Figuren 4A und 4B näher beschrieben wird. Beim Eindringen eines Aktors in die Aktoröffnung 126 kann ebenfalls wiederum die Versiegelung 122 auf der Oberschale 116 perforiert werden.

Nachdem die Probe auf das Testfeld 136 übertragen ist, kann von der Unterseite des analytischen Magazins 110 her beispielsweise eine Farbänderung des Testfeldes 136 im Bereich der Öffnung 158 erfolgen, beispielsweise mittels entsprechender optischer Detektoren, Beleuchtungen oder ähnlichen dem Fachmann bekannten Elementen.

An die Analyse anschließend, dieser vorgelagert oder gleichzeitig zur Analyse kann wiederum ein Abkoppeln der Aktorstange von dem Koppelelement 172 erfolgen, indem beispielsweise die Lanzette 164 wieder soweit in der Kammer 112 zurückgezogen wird, dass sich das Koppelelement 172 aus der Lanzettenebene der Lanzette 164 heraus biegt und so vom Haken der Aktorstange gelöst wird. Die Aktorstange kann die Kammer 112 dann durch die Innenöffnung 122 wieder verlassen.

Bei der in Figur 1 gezeigten Zusammenbaudarstellung des analytischen Magazins 110 ist erkennbar, dass das gezeigte analytische Magazin 110 im dargestellten Beispiel 50 analytische Hilfsmittel 162 umfasst. Wie beispielsweise aus Figur 2E erkennbar ist, können die Lanzetten beispielsweise aus einer gemeinsamen Metallscheibe 176 herausgeätzt sein, und mit dieser Metallscheibe 176 noch beispielsweise durch Stege oder anderen Verbindungselementen verbunden sein. Entsprechend können die Lanzetten 164 gemeinsam in die Vertiefungen 144 in der Unterschale 118 eingesetzt werden. Anschließend können die Lanzetten 164 durch einen entsprechenden Prozess aus der Metallscheibe 176 gleichzeitig oder nacheinander herausgebrochen werden, so dass das Einfügen der Lanzetten 164 allgemein gleichzeitig in die einzelnen Kammern 112 erfolgen kann.

Die Membran 128, welche im dargestellten Beispiel als Membranring 130 für alle Kammern 112 gleichzeitig ausgestaltet ist, kann im Wesentlichen homogen ausgestaltet sein und kann für mehrere Kammern oder, wie in den Figuren dargestellt, für alle Kammern jeweils mindestens eine Durchtrittsfläche 178 bereitstellen, durch welche flüssige Probe aus dem Inneren der Kammer 112 zur zugehörigen Testfeldfläche des Testfeldes 136 gelangen kann. Die Membran 128 kann jedoch, wie ebenfalls in den Figuren 2C und 3 angedeutet, auch strukturiert ausgestaltet sein und neben den Durchtrittsflächen 178, welche beispielsweise im Wesentlichen den Öffnungen 158 der Testelementfenster 160 entsprechen können, für die Probe der Körperflüssigkeit undurchlässige Flächen 180 umfassen.

Das analytische Magazin 110 in dem in den Figuren 1 bis 3 dargestellten Ausführungsbeispiel ist also als scheibenförmiges Magazin in Form einer Kreisscheibe ausgestaltet und enthält in diesem Ausführungsbeispiel 50 analytische Hilfsmittel 162. Die Herstellung ist kostengünstig und erfordert in der Regel keine biokompatible Testchemie 140 oder stellt erheblich geringere Anforderungen an eine Biokompatibilität der Testchemie 140, da durch die Membran 128 ein Eindringen von Testchemie 140 ins Innere der Kammern 112 verhindert werden kann. Die Membran 128 kann somit im Wesentlichen semipermeabel ausgestaltet sein und zwischen die Unterschale 118 und den Chemiering 138 platziert werden. Die Membran 128 kann insbesondere porös ausgestaltet werden, mit derart kleinen Poren, dass diese keimdicht ist und im Wesentlichen keinen Chemiestaub der Testchemie 140 durchlässt. Andererseits lässt es sie bei der Benetzung mit Flüssigkeit, beispielsweise Blut, Bestandteile der Flüssigkeit, beispielsweise Plasma und Glukose, durchtreten.

Der Innenraum der Kammern 112 ist also doppelt gegenüber Verunreinigungen durch partikuläre Kontamination und Keime geschützt. Zum einen sind durch die Kanten der Öffnungen 158 in den Wänden 156 die Schneidekanten 142 des Testchemierings 138, welche besonders partikelanfällig sind, abgedeckt. Andererseits wird durch die Membran 128 ein weiterer Schutz des Innenraums der Kammern 112 gegenüber Keimen und Chemiestaub gewährleistet.

In einer Fertigung kann, beispielsweise nach einem Verbinden der Oberschale 116 und der Unterschale 118, beispielsweise durch ein Laserschweißverfahren, und gegebenenfalls nach Aufbringen der Versiegelung 120, zunächst beispielsweise der Membranring 130 in die Ringnut 132 in der Unterschale 118 eingebracht und beispielsweise durch Warmverpressen oder ähnliche Verbindungstechniken mit der Unterschale 118 beispielsweise Stegen dieser Unterschale 118, verbunden werden. Gleichzeitig mit dieser Verbindung kann die in Figur 2C angedeutete Strukturierung erfolgen. Alternativ oder zusätzlich kann die angedeutete Strukturierung in Durchtrittsflächen 178 und undurchlässige Flächen 180 auch separat erfolgen, was jedoch eine Positionierung des Membranrings 130 relativ zu den Öffnungen 158 erforderlich macht. Außerhalb der Einzelfelder der Durchtrittsflächen 178 kann die Membran 128, beispielsweise durch thermische Einwirkung, derart verdichtet werden, dass die undurchlässige Fläche 180 in Form einer verdichteten Folie entsteht, welche beispielsweise keine Flüssigkeit mehr aufnimmt. Auch eine lokale Laserbehandlung kann beispielsweise die Struktur zu einer Folie verdichten. Lediglich in den unverdichteten Feldern der Durchtrittsflächen 178 bleibt ihre Membranfunktion dann noch erhalten. Eine Feldgröße der Durchtrittsflächen 178 kann beispielsweise bei ca. 0,5 mm x 0,5 mm liegen.

Nach diesem Einbringen des Membranrings 130 kann dann, sofern dies nicht vorher bereits erfolgt ist, eine Verbindung der übrigen Bestandteile erfolgen, und es können beispielsweise die Unterschale 118 in Form des Bodenrads, die Microsampler 166 und die Oberschale 116 in Form des Deckelrades zusammengefügt und gegebenenfalls verbunden werden.

Ein derartig erzeugtes Halbfabrikat kann dann beispielsweise sterilisiert und anschließend gelagert werden. Die Membran 128 erhält dabei die Sterilität des Innenraums der Kammern 112. Zur Fertigstellung muss dann das Halbfabrikat lediglich mit der Testchemie 140 ausgestattet werden. Hierzu kann der Testchemiering 138 in die Ringnut 132 eingeclipst werden. Zu diesem Zweck können beispielsweise am Rand der Ringnut 132 Haltenasen und/oder andere Verbindungselemente, beispielsweise ein vorspringender Ring, angeordnet sein. Der Testchemiering 138 liegt dann vorzugsweise eben und insbesondere unmittelbar auf dem Membranring 130 auf.

Die Membran 128 kann, neben den oben genannten Funktionen, weitere Funktionen erfüllen. So kann diese beispielsweise eine Spreitfunktion erfüllen. So kann die Membran 128 beispielsweise ausgestaltet sein, um die Probe der Körperflüssigkeit, beispielsweise das Blut, nicht einfach vertikal zum Testfeld 136 hindurchzuleiten, sondern diese aufzuspreiten auf das gesamte, beispielsweise durch die zugehörige Öffnung 158 definierte Teil-Testfeld bzw. die Testfeldoberfläche des Testfeldes 136, welche der Kammer 112 zugeordnet ist. Daher ist das benetzte Feld auf dem Testfeld 136 ebenfalls verbreitert, und die Auswertung wird mit einer einfacheren Optik möglich. Es kann dann entweder eine weniger hoch auflösende Optik verwendet werden, oder es kann, alternativ oder zusätzlich, auch eine miniaturisierte Lichtleiteroptik und/oder eine konventionelle Optik verwendet werden, wobei letztere vorzugsweise sorgfältig an Radstrukturen geführt werden kann, damit diese präzise die relevanten Bereiche vermessen kann.

Wegen der Spreitung der Probe sollte der Kapillarspalt 170 bzw. eine andere Form von Kapillare auf einer Länge befüllt sein, welche größer ist als die Länge des durch die Öffnung 158 und/oder die Durchtrittsfläche 178 definierten Bereichs des Testfeldes 136, also die der Kammer 112 zuweisende bzw. von der Kammer 112 aus zugängliche Testfeldoberfläche. Weist beispielsweise der Kapillarspalt 170 eine Länge von 2 mm auf, so kann bei einem Membranfeld in Form einer Durchtrittsfläche 178 mit einer Länge von 0,5 mm eine seitliche Spreitung um ca. einen Faktor 4 erzielt werden, da die Membran 128 durch Saugkräfte die Flüssigkeit aus der dem Kapillarspalt 170 aufnimmt. Diese Angaben gelten unter der Annahme, dass der Kapillarspalt 170 und die Membran 128 eine im Wesentlichen gleiche Blutfüllhöhe aufweisen, was bei ca. 80 µm Kanaltiefe des Kapillarspalts 170 und einer Membrandicke von beispielsweise ca. 100 µm im Wesentlichen erfüllt ist. So kann beispielsweise ein benetztes Feld von ca. 0,5 mm x 0,5 mm auf dem Testfeld 136 erzeugt werden.

In dem in den Figuren 1 bis 3 dargestellten Ausführungsbeispiel des analytischen Magazins 110 erfolgt also, wie oben beschrieben, ein Übertrag einer Probe der Körperflüssigkeit vom Microsampler 166 auf das Testfeld 136 durch einen separaten Aktorschritt, bei welchem ein Teil des Aktors durch die Aktoröffnung 126 ins Innere der Kammer 112 eindringt und den Microsampler 166 dem Testfeld 136 annähert. Auch andere Mechanismen sind möglich. Ein weiteres Ausführungsbeispiel eines analytischen Magazins 110 ist in den Figuren 4A und 4B gezeigt, welches einen anderen Mechanismus eines Übertrags einer von einem Microsampler 166 aufgenommenen Probe 182 einer Körperflüssigkeit auf das Testfeld 136 realisiert. Dabei zeigen die Figuren 4A und 4B jeweils Schnittdarstellungen von der Seite durch eine Kammer 112 eines analytischen Magazins 112, welches beispielsweise ansonsten im Wesentlichen analog zum Ausführungsbeispiel in den Figuren 1 bis 3 ausgestaltet sein kann.

Zunächst ist erkennbar, dass wiederum am Boden der Kammern 112, in der Unterschale 118, Öffnungen 158 ausgebildet sind, beispielsweise wiederum rechteckige Testelementfenster 160. Diese Öffnungen 158 definieren auf dem Testfeld 136 in Form des Testchemierings 138 wiederum zugängliche Testfeldflächen, welche hier mit der Bezugsziffer 184 bezeichnet sind. Diese zugänglichen Testfeldflächen 184 bilden somit Teile des analytischen Hilfsmittels 162, da diese wie Einzel-Testfelder wirken, analog zu den Ausführungsformen in den Figuren 1 bis 3. Wiederum überdecken die Wände 156 der Kammern 112 dabei die Schneidekanten 142 des Testchemierings 138, so dass wiederum weitgehend auf die obige Beschreibung verwiesen werden kann.

Außerdem weist auch in den Figuren 4A und 4B eine zwischen das Testfeld 136 und den Innenraum der Kammern 112 eingebrachte Membran 128 in Form eines Membranrings 130 wiederum, analog zum obigen Ausführungsbeispiel, Durchtrittsflächen 178 und undurchlässige Flächen 180 auf. Die undurchlässigen Flächen 180 können wiederum beim Verbinden der Membran 128 mit der Unterschale 118 und/oder in einem separaten Schritt erzeugt werden, beispielsweise durch ein entsprechendes Verdichten, und/oder in einem separaten Schritt. Somit kann die zugängliche Testfeldfläche 184 für jede Kammer 112 auch, alternativ zu einer Definition durch die Kanten der Öffnungen 158, durch die Durchtrittsflächen 178 definiert werden, welche auch abweichend von den Öffnungen 158 gestaltet werden können, beispielsweise größer oder kleiner.

Zum Übertrag der Probe 182 auf das Testfeld 136 wird in den Figuren 4A und 4B ein Mechanismus vorgeschlagen, bei welchem der Microsampler 166 in mindestens einer Position auf der Membran 128 aufliegt. Ein derartiger Mechanismus ist bei Testelementen 134 ohne Membran 128 nicht empfehlenswert, da ein derartiges Aufliegen zu einem mechanischen Abrieb und zu einem unmittelbaren Übertrag von möglicherweise allergieauslösenden Bestandteilen der Testchemie 140 auf den Microsampler 166 führen kann. Durch die erfindungsgemäße Ausgestaltung mit der Membran 128 kann dies jedoch nahezu ausgeschlossen werden, da die Membran 128 entsprechende Partikel zurückhält.

Der Microsampler 166 ist in den Figuren lediglich angedeutet. In Figur 4A ist dabei eine unbenutzte Kammer 112 dargestellt, in welche der Microsampler 166 beispielsweise in einer Lagerposition 186 angeordnet ist. Die Versiegelungen 120 an den Öffnungen 122, 124 sind in diesem Zustand noch unversehrt. In Figur 4B ist hingegen eine Situation gezeigt, in welcher die oben beschriebene Probennahmebewegung durchgeführt ist und in welche der Microsampler 166 in der Kammer 112 remagaziniert ist. Der Innenraum der Kammer 112 ist derart gekrümmt ausgeführt, dass der Microsampler 166 mit der Lanzettenspitze 168 eine gekrümmte Bahn beschreibt. Wird der Microsampler 166, beispielsweise durch einen Aktor des Systems, zurückgezogen (in Figur 4B mit den Pfeilen 190 bezeichnet), so kommt in mindestens einer, in Figur 4B mit der Bezugsziffer 188 bezeichneten Übertragungsposition, die Lanzettenspitze 168 auf der Membran 128 zu liegen. Nach dem Stich kann dann die gefüllte Lanzette 164 beispielsweise automatisch, auf der Membran 128 aufliegen, so dass diese Membran 128 die flüssige Probe 182 aufsaugen kann. Das Überbrücken eines Spaltes zwischen Microsampler 166 durch den Druck eines Aktors, beispielsweise eines Stößels, auf die Lanzette 164, analog zu dem Ausführungsbeispiel in den Figuren 1 bis 3, ist in diesem Fall nicht erforderlich. Somit kann ein Aktor des analytischen Systems erheblich vereinfacht werden, und es kann eine Versiegelung 120 der Aktoröffnungen 126 mit samt den Aktoröffnungen 126 selbst eingespart werden.

Alternativ oder zusätzlich kann der Kontakt des Testfeldes 136 mit der Membran 128 und/oder dem Microsampler 166 zusätzlich erhöht werden, beispielsweise indem von unten auf das Testfeld 136 Druck abgesenkt wird. So kann beispielsweise in einem letzten Teil des Zurückziehens 190 des Microsamplers 166 die gesamte Scheibe des analytischen Magazins 110 abgesenkt werden und mit dem Chemiering 138 auf eine Optik oder ein ähnliches Anpresselement gedrückt werden.

Soll das Aufliegen der Lanzette 164 und insbesondere der des Kapillarspalts 170 auf der Membran 128 während einer längeren Lagerzeit vermieden werden, beispielsweise um eine Beschädigung der Membran 128 durch die Lanzette 164 und/oder eine Kontamination der Lanzette 164 durch die Membran 128 zu vermeiden, so kann, wie in Figur 4A gezeigt, optional die Lagerposition 186 vorgesehen sein. Diese kann beispielsweise derart ausgestaltet sein, dass in dieser Position die Lanzette 164 etwas weiter außen platziert ist, wo sie auf dem Rand der Unterscheibe 118 leicht erhöht und beispielsweise leicht gebogen aufliegt. Nach dem Stich, also nach der Probennahmebewegung, kann dann die Lanzette 164, wie in Figur 4B gezeigt, etwas weiter zurückgezogen werden, die Vorbiegung entspannt sich, und der Microsampler 166 mit dem gefüllten Kapillarspalt 170 kann die Membran 128 berühren. Auf diese Weise kann auch das Absenken der Scheibe, welches oben als Option beschrieben wurde, vermieden werden, und ganz oder teilweise durch die Durchbiegung der Lanzette 164 ersetzt werden. Es sei darauf hingewiesen, dass in den Figuren 4A und 4B die Lanzette 164 lediglich schematisch angedeutet ist, und dass diese zusätzliche Elemente umfassen kann, beispielsweise einen Lanzettenschaft, beispielsweise analog zur Darstellung in Figur 3.

Allgemein wird darauf hingewiesen, dass das analytische Magazin 110 eine Reihe weiterer, in den Figuren nicht dargestellte Elemente, umfassen kann. Beispielsweise können, wie in Figur 1 auf der Oberschale 116 angedeutet, Transport- und Positionierungselemente 192 vorgesehen sein, welche es beispielsweise einem in einer Innenöffnung 194 des analytischen Magazins angeordneten Transportmechanismus ermöglichen, jeweils exakt eine Kammer 112 in einer Applikationsposition eines analytischen Systems zu positionieren.

### Bezugszeichenliste

- 110: analytisches Magazin
- 112: Kammer
- 114: Gehäuse
- 116: Oberschale
- 118: Unterschale
- 120: Versiegelung
- 122: Innenöffnungen
- 124: Außenöffnungen
- 126: Aktoröffnungen
- 128: Membran
- 130: Membranring
- 132: Ringnut
- 134: Testelemente
- 136: Testfeld
- 138: Testchemiering
- 140: Testchemie
- 142: Schneidekanten
- 144: Vertiefungen
- 156: Wände der Kammern
- 158: Öffnungen
- 160: Testelementfenster
- 162: analytische Hilfsmittel
- 164: Lanzette
- 166: Microsampler
- 168: Lanzettenspitze
- 170: Kapillarspalte
- 172: Koppelelemente
- 174: Aktornut
- 176: Metallscheibe
- 178: Durchtrittsfläche
- 180: undurchlässige Fläche
- 182: Probe
- 184: zugängliche Testfeldflächen
- 186: Lagerposition
- 188: Übertragungsposition
- 190: Zurückziehen
- 192: Transport- und Positionierungselemente
- 194: Innenöffnung

## Patentansprüche

1. Analytisches Magazin (110), umfassend mindestens eine Kammer (112) mit mindestens einem analytischen Hilfsmittel (162), wobei das analytische Hilfsmittel (162) mindestens ein Testelement (134) zum Nachweis mindestens eines Analyten in einer Probe (182) einer Körperflüssigkeit umfasst, wobei das Testelement (134) mindestens ein Testfeld (136) mit mindestens einer Testchemie (140) umfasst, **dadurch gekennzeichnet, dass** zwischen dem Testfeld (136) und einem Innenraum der Kammer (112) zumindest eine Membran (128) angeordnet ist, wobei die Membran (128) zumindest teilweise durchlässig für die Probe (182) ist, insbesondere für Blut oder Blutbestandteile, wobei die Membran (128) weiterhin zumindest teilweise undurchlässig ist für die Testchemie (140), so dass ein Eindringen von Bestandteilen der Testchemie in die Kammer (112) im Wesentlichen verhindert wird, wobei feste Chemiebestandteile mit einer Größe von mehr als 10 Mikrometern zurückgehalten werden, wobei die Membran (128) ganz oder teilweise als poröses, für die Probe und/oder Bestandteile der Probe durchlässiges Material ausgestaltet ist, wobei die Membran (128) unmittelbar auf dem Testfeld (136) aufliegt, so dass zwischen dem Testfeld (136) und der Membran (128) kein Zwischenraum entsteht oder ein Zwischenraum von 5µm oder weniger.

2. Analytisches Magazin (110) nach dem vorhergehenden Anspruch, wobei das Testfeld (136) von der Membran (128) zumindest teilweise abgedeckt wird.

3. Analytisches Magazin (110) nach einem der beiden vorhergehenden Ansprüche, wobei die Membran (128) eine mittlere Porengröße zwischen 1 µm und 10 µm, vorzugsweise zwischen 2 µm und 5 µm, aufweist.

4. Analytisches Magazin (110) nach einem der drei vorhergehenden Ansprüche, wobei die Membran (128) eine Dicke zwischen 30 µm und 150 µm, insbesondere zwischen 50 µm und 100 µm, aufweist.

5. Analytisches Magazin (110) nach einem der vier vorhergehenden Ansprüche, wobei die Membran (128) eines oder mehrere der folgenden Materialien aufweist: eine Zellulose, insbesondere eine Nitrozellulose; ein Polysulfon; ein Nylon; ein Polyvinylidenfluorid; eine regenerierte Zellulose; ein hydrophiles Polyurethan.

6. Analytisches Magazin (110) nach einem der fünf vorhergehenden Ansprüche, wobei die Membran (128) mindestens eine Strukturierung aufweist, mit mindestens einer Durchtrittsfläche (178), welche für die Probe (182) der Körperflüssigkeit zumindest teilweise durchlässig ist, und mindestens einer undurchlässigen Fläche (180), welche für die Probe (182) der Körperflüssigkeit zumindest teilweise undurchlässig ist.

7. Analytisches Magazin (110) nach dem vorhergehenden Anspruch, wobei das analytische Magazin (110) eine Mehrzahl von Kammern (112) aufweist, wobei die Membran (128) für mehrere Kammern (112), vorzugsweise für alle Kammern (112), gemeinsam ausgebildet ist und für die mehreren Kammern (112), insbesondere für alle Kammern (112), jeweils mindestens eine Durchtrittsfläche (178) bereitstellt.

8. Analytisches Magazin (110) nach einem der sieben vorhergehenden Ansprüche, wobei das analytische Magazin (110) ein Gehäuse (114) mit mindestens einer der Kammer (112) zuweisenden Öffnung (158) aufweist, wobei die Membran (128) von außen auf die Öffnung (158) aufgebracht ist und durch die Öffnung (158) von der Kammer (112) aus zugänglich ist.

9. Analytisches Magazin (110) nach dem vorhergehenden Anspruch, wobei die Membran (128) mit dem Gehäuse (114) verbunden ist durch mindestens eine Verbindung, welche eine oder mehrere der folgenden Verbindungen umfasst: eine Schweißverbindung, insbesondere eine thermische Schweißverbindung und/oder eine warmgestemmte Verbindung, eine Ultraschall-Schweißverbindung, eine Laser-Schweißverbindung; eine Klebeverbindung.

10. Analytisches Magazin (110) nach einem der beiden vorhergehenden Ansprüche, wobei das Testfeld (136) mit dem Gehäuse (114) verbunden ist durch mindestens eine kraftschlüssige und/oder formschlüssige Verbindung, insbesondere eine Clip-Verbindung.

## Claims

1. Analytic magazine (110), comprising at least one chamber (112) with at least one analytic aid (162), wherein the analytic aid (162) comprises at least one test element (134) for detecting at least one analyte in a sample (182) of a bodily fluid, wherein the test element (134) comprises at least one test field (136) with at least one test chemical (140), **characterized in that** at least one membrane (128) is arranged between the test field (136) and an interior space of the chamber (112), wherein the membrane (128) is at least partly permeable to the sample (182), more particularly to blood or blood constituents, wherein the membrane (128) furthermore is at least partly impermeable to the test chemical (140) such that an ingress of components of the test chemical into the chamber (112) is substantially prevented, wherein solid chemical components with a size of more than 10 micrometers are held back, wherein the membrane (128) is wholly or partly embodied as a porous material that is permeable to the sample and/or components of the sample, wherein the membrane (128) lies directly on the test field (136) such that no interspace, or an interspace of 5 µm or less, is produced between the test field (136) and the membrane (128).

2. Analytic magazine (110) according to the preceding claim, wherein the test field (136) is at least partly covered by the membrane (128).

3. Analytic magazine (110) according to one of the two preceding claims, wherein the membrane (128) has a mean pore dimension of between 1 µm and 10 µm, preferably of between 2 µm and 5 µm.

4. Analytic magazine (110) according to one of the three preceding claims, wherein the membrane (128) has a thickness of between 30 µm and 150 µm, more particularly between 50 µm and 100 µm.

5. Analytic magazine (110) according to one of the four preceding claims, wherein the membrane (128) has one or more of the following materials: a cellulose, more particularly a nitrocellulose, a polysulfone, a nylon, a polyvinylidene fluoride, a regenerated cellulose, a hydrophilic polyurethane.

6. Analytic magazine (110) according to one of the five preceding claims, wherein the membrane (128) has at least one structuring with at least one passage area (178), which is at least partly permeable to the sample (182) of the bodily fluid, and with at least one impermeable area (180) that is at least partly impermeable to the sample (182) of the bodily fluid.

7. Analytic magazine (110) according to the preceding claim, wherein the analytic magazine (110) has a plurality of chambers (112), wherein the membrane (128) is embodied in common for a number of chambers (112), preferably for all chambers (112), and in each case provides at least one passage area (178) for the number of chambers (112), more particularly for all chambers (112).

8. Analytic magazine (110) according to one of the seven preceding claims, wherein the analytic magazine (110) has a housing (114) with at least one opening (158) facing the chamber (112), wherein the membrane (128) is applied to the opening (158) from the outside and is accessible from the chamber (112) through the opening (158).

9. Analytic magazine (110) according to the preceding claim, wherein the membrane (128) is connected to the housing (114) by at least one connection, which comprises one or more of the following connections: a welded joint, more particularly a thermal welded joint and/or a connection produced by heat staking, an ultrasound welded joint, a laser welded joint; a bonded connection.

10. Analytic magazine (110) according to one of the two preceding claims, wherein the test field (136) is connected to the housing (114) by at least one force-fit and/or form-fit connection, more particularly a clip-on connection.

## Revendications

1. Magasin d'analyse (110), comprenant au moins une chambre (112) avec au moins un accessoire d'analyse (162), dans lequel l'accessoire d'analyse (162) comprend au moins un élément de test (134) pour rechercher au moins un analyte dans un échantillon (182) d'un liquide corporel, dans lequel l'élément de test (134) comprend au moins un champ de test (136) avec au moins une chimie de test (140), **caractérisé en ce qu'**au moins une membrane (128) est disposée entre le champ de test (136) et un espace intérieur de la chambre (112), dans lequel la membrane (128) est au moins partiellement perméable pour l'échantillon (182), en particulier pour le sang ou des composants du sang, dans lequel la membrane (128) est en outre au moins partiellement imperméable pour la chimie de test (140), de telle manière qu'une pénétration de composants de la chimie de test dans la chambre (112) soit essentiellement empêchée, dans lequel les composants solides de la chimie avec une taille de plus de 10 micromètres sont retenus, dans lequel la membrane (128) est réalisée entièrement ou partiellement sous forme de matériau poreux, perméable pour l'échantillon et/ou des composants de l'échantillon, dans lequel la membrane (128) repose directement sur le champ de test (136), de telle manière qu'il n'existe aucun espace intermédiaire entre le champ de test (136) et la membrane (128) ou un espace intermédiaire de 5 µm ou moins.

2. Magasin d'analyse (110) selon la revendication précédente, dans lequel le champ de test (136) est recouvert au moins en partie par la membrane (128).

3. Magasin d'analyse (110) selon l'une des deux revendications précédentes, dans lequel la membrane (128) présente une taille de pore moyenne comprise entre 1 µm et 10 µm, de préférence entre 2 µm et 5 µm.

4. Magasin d'analyse (110) selon l'une des trois revendications précédentes, dans lequel la membrane (128) présente une épaisseur comprise entre 30 µm et 150 µm, en particulier entre 50 µm et 100 µm.

5. Magasin d'analyse (110) selon l'une des quatre revendications précédentes, dans lequel la membrane (128) présente un ou plusieurs des matériaux suivants: une cellulose, en particulier une nitrocellulose; un polysulfone; un nylon; un fluorure de polyvinylidène; une cellulose régénérée; un polyuréthane hydrophile.

6. Magasin d'analyse (110) selon l'une des cinq revendications précédentes, dans lequel la membrane (128) présente au moins une structuration, avec au moins une face de passage (178), qui est au moins partiellement perméable pour l'échantillon (182) du liquide corporel, et au moins une face imperméable (180), qui est au moins partiellement imperméable pour l'échantillon (182) du liquide corporel.

7. Magasin d'analyse (110) selon la revendication précédente, dans lequel le magasin d'analyse (110) présente une multiplicité de chambres (112), dans lequel la membrane (128) est commune pour plusieurs chambres (112), de préférence pour toutes les chambres (112), et procure respectivement au moins une face de passage (178) pour plusieurs chambres (112), en particulier pour toutes les chambres (112).

8. Magasin d'analyse (110) selon l'une des sept revendications précédentes, dans lequel le magasin d'analyse (110) présente un boîtier (114) avec au moins une ouverture (158) orientée vers la chambre (112), dans lequel la membrane (128) est placée sur l'ouverture (158) par l'extérieur et est accessible à partir de la chambre (112) à travers l'ouverture (158).

9. Magasin d'analyse (110) selon la revendication précédente, dans lequel la membrane (128) est assemblée au boîtier (114) par au moins un assemblage, qui comprend un ou plusieurs des assemblages suivants: un assemblage soudé, en particulier un assemblage soudé à chaud et/ou un assemblage rabattu à chaud, un assemblage soudé aux ultrasons, un assemblage soudé au laser, un assemblage collé.

10. Magasin d'analyse (110) selon une des deux revendications précédentes, dans lequel le champ de test (136) est assemblé au boîtier (114) par au moins un assemblage par adhérence et/ou par emboîtement, en particulier un assemblage par encliquetage.
